# EUROPÄISCHE PATENTSCHRIFT

(11) **EP 4 197 580 B1**
(45) Veröffentlichungstag und Bekanntmachung des Hinweises auf die Patenterteilung: **12.03.2025**
(21) Anmeldenummer: 22212762.3
(22) Anmeldetag: 12.12.2022
(51) Int. Cl.: A61M 16/12, A61M 16/01

(54) **ANORDNUNG ZUR VERSORGUNG EINER PATIENTENSEITIGEN KOPPELEINHEIT MIT EINEM GASGEMISCH**
ARRANGEMENT FOR SUPPLYING A PATIENT-SIDE COUPLING UNIT WITH A GAS MIXTURE
SYSTÈME POUR ALIMENTER UN ENSEMBLE DE COUPLAGE CÔTÉ PATIENT AVEC UN MÉLANGE GAZEUX

(30) Priorität: 14.12.2021 DE 102021132928
(43) Veröffentlichungstag der Anmeldung: 21.06.2023
(73) Patentinhaber: Drägerwerk AG & Co. KGaA, 23558 Lübeck (DE)
(72) Erfinder: Hansmann, Hans-Ullrich, 23558 Lübeck (DE); Garbrecht, Oliver, 23558 Lübeck (DE); Fischer, Hendrik, 23558 Lübeck (DE)

(56) Entgegenhaltungen:
- WO-A1-2021/203050
- US-A- 3 664 361
- US-A- 3 762 427
- US-A- 4 549 563

## Beschreibung

Die Erfindung betrifft eine Versorgungs-Anordnung, um eine patientenseitige Koppeleinheit mit einem Gasgemisch zu versorgen.

Die Erfindung lässt sich beispielsweise für die künstliche Beatmung eines Patienten verwenden. Im oder am Körper des Patienten ist eine patientenseitige Koppeleinheit angeordnet, beispielsweise eine Atemmaske, ein Katheter oder ein Tubus. Um den Patienten künstlich zu beatmen, wird ein Gasgemisch zu der patientenseitigen Koppeleinheit gefördert. Dieses Gasgemisch umfasst Sauerstoff und in einer Ausgestaltung zusätzlich mindestens ein Narkosemittel. Bevorzugt führt ein Beatmungsgerät eine Abfolge von Beatmungshüben aus und fördert in jedem Beatmungshub eine Menge des Gasgemischs zur patientenseitigen Koppeleinheit.

Möglich ist, dass als Gasgemisch Atemluft verwendet wird. Oft wird gewünscht, dass der Anteil von Sauerstoff in dem Gasgemisch, welches zur patientenseitigen Koppeleinheit gefördert wird, größer als der Sauerstoffanteil in der Atemluft ist. Um dieses Ziel zu erreichen, wird ein Gasgemisch umfassend Atemluft und reinem Sauerstoff erzeugt. Die Erfindung lässt sich dafür anwenden, um ein solches Gasgemisch zu erzeugen und zur patientenseitigen Koppeleinheit zu fördern.

Eine Versorgungs-Anordnung mit den Merkmalen des Oberbegriffs des Anspruchs 1 ist aus US 3 664 361 A bekannt. Dort wird eine Beatmungs-Anordnung (respiration system) beschrieben, die Sauerstoff von einer druckgeregelten Quelle (pressure regulated source 10) mit Luft von einem Lufteinlass (air intake 11) vermischt und das Ergebnis der Vermischung an einen Patienten leitet. Die Luft wird mittels einer Pumpe 13 vom Einlass 11 über eine erste Leitung 14, einem Bauteil (manifold 19) und einer zweiten Leitung 15 zu einer Mischeinheit (proportioning device 20) gefördert. Der Sauerstoff wird über Leitungen 41, 53, 32 zur Mischeinheit 20 gefördert. Ein mechanischer Regler (control apparatus 33) passt den Druck des Sauerstoffs in der Leitung 32 an abgetastete Schwankungen des Drucks in der Leitung 14 an. Der Regler 33 umfasst ein Reservoir 35 mit variablem Volumen, wobei das Reservoir 35 eine feste Wand 43, eine bewegliche Wand 45 und zwei faltbare Wände 47 umfasst und wobei im Reservoir 35 ein Überdruck gegenüber der Umgebung herrscht. Ein Ausgleichsventil (balancing valve 37) öffnet und schließt eine Ausgleichsöffnung (balancing port 38) in einer Wand 43 zwischen dem Reservoir 35 und der Leitung 32. Eine Membran (diaphragm 39) ist auf der einen Seite dem Druck in der Leitung 32 ausgesetzt und auf der anderen Seite einem Druck, der über eine Leitung 79 dem Luftdruck in der Leitung 14 ausgesetzt ist.

Die Beatmungs-Anordnung von US 4 549 563 A fördert ein Gasgemisch umfassend Sauerstoff aus einer Quelle 11 und Stickstoff aus einer Quelle 21 und optional ein Narkosemittel zu einer Atemmaske auf dem Gesicht eines Patienten. Die Bestandteile des Gasgemisch werden in einer Mischeinheit (circuit 3) miteinander vermischt. Ein Druckminderer (pressure limiter 40) umfasst zwei Kammern 42 und 43 sowie eine Membrane (diaphragm 41) zwischen den beiden Kammern 42 und 43. Die Kammer 42 ist mit der Quelle 21 und mit einer Leitung 20 verbunden, wobei die Leitung 20 zur Mischeinheit 3 führt. Die Kammer 43 ist über eine Leitung 46 mit einer Leitung 10 verbunden, wobei die Leitung 10 die Quelle 11 mit der Mischeinheit 3 verbindet.

Das Beatmungsgerät 14 von EP 2 425 869 A1 umfasst eine Mischvorrichtung und einen Ventilationsteil. In der Mischvorrichtung wird ein medizinisches Gas, beispielsweise Sauerstoff oder ein Narkosemittel, mit Luft vermischt. Das medizinische Gas wird über einen Gaseinlass B eingespeist und über eine Gasleitung 10 zugeführt. Die Luft wird über einen Lufteinlass A eingespeist und über eine Luftleitung 11 zugeführt. In der Gasleitung 10 sind ein Reduktionsventil 6, ein Sicherheitsventil 7, ein ansteuerbares Proportionalventil 8 und ein Durchflussmesser 9 angeordnet. In der Luftleitung 11 sind ein Gebläse 1 und ein Rückschlagventil 2 angeordnet. Der Ventilationsteil leitet das Gasgemisch über eine Atemgasleitung 12 einem Patienten als Atemgas zu. In der Atemgasleitung 12 sind ein Durchflusssensor 3, ein ansteuerbares Proportionalventil 4 und ein Drucksensor 5 angeordnet. Eine Steuereinheit 13 empfängt Signale von den Sensoren 9, 3 und vermag alle ansteuerbaren Ventile 8, 4 anzusteuern.

Der Erfindung liegt die Aufgabe zugrunde, eine Versorgungs-Anordnung bereitzustellen, welche eine patientenseitige Koppeleinheit mit einem Gasgemisch umfassend mindestens zwei Gas-Bestandteile zu versorgen vermag, wobei der zeitliche Verlauf des Drucks des bereitgestellten Gasgemischs oder des Volumenflusses des Gasgemischs zur patientenseitigen Koppeleinheit sich relativ betriebssicher regeln lässt.

Die Erfindung wird durch eine Versorgungs-Anordnung mit den Merkmalen des Anspruchs 1 gelöst. Vorteilhafte Ausgestaltungen sind in den Unteransprüchen angegeben.

Die erfindungsgemäße Versorgungs-Anordnung vermag eine patientenseitige Koppeleinheit mit einem Gasgemisch zu versorgen. Das Gasgemisch umfasst einen ersten Gas-Bestandteil, beispielsweise Atemluft, und einen zweiten Gas-Bestandteil, beispielsweise reinen Sauerstoff. Das Gasgemisch kann einen dritten Gas-Bestandteil umfassen, beispielsweise ein Narkosemittel.

Die patientenseitige Koppeleinheit ist mit einem Patienten verbunden oder lässt sich wenigstens zeitweise mit einem Patienten verbinden. Insbesondere lässt die patientenseitige Koppeleinheit sich auf dem Gesicht des Patienten anordnen und / oder in den Körper des Patienten einführen.

Nachfolgend wird der Begriff "Kanal" verwendet. Unter einem Kanal wird ein Bauteil verstanden, welches ein Fluid, insbesondere ein Gas oder Gasgemisch, entlang einer vorgegebenen Trajektorie zu führen vermag und idealerweise verhindert, dass das Fluid diese Trajektorie verlässt. Ein Schlauch und eine Röhre sind Beispiele für einen Kanal.

Außerdem ist nachfolgend davon die Rede, dass eine Fluidverbindung zwischen zwei Bauteilen hergestellt ist. Darunter wird verstanden, dass ein Fluid vom einen Bauteil zum anderen Bauteil fließen kann, idealerweise ohne in die Umgebung zu entweichen. Möglich ist, dass die beiden Bauteile direkt miteinander verbunden sind. Möglich ist auch, dass ein Abstand zwischen den beiden Bauteilen auftritt und eine Fluidführungseinheit, beispielsweise ein Schlauch, die beiden Bauteile miteinander verbindet. Möglich ist, dass ein Fluid zeitweise vom ersten Bauteil durch die Fluidverbindung hindurch zum zweiten Bauteil und zeitweise umgekehrt vom zweiten Bauteil durch die Fluidverbindung hindurch zum ersten Bauteil fließt. Die Fluidverbindung kann dauerhaft oder nur zeitweise hergestellt sein.

Unter einem "Pufferspeicher" wird im Kontext der Erfindung ein Bauteil verstanden, welches in seinem Inneren eine Menge eines Fluids aufnehmen und wieder abgeben kann. Möglich ist, dass das Volumen des Pufferspeichers sich verändert, wobei der Pufferspeicher sich bei Aufnahme des Gases vergrößert und bei Abgabe des Gases wieder verkleinert. Möglich ist auch, dass das Volumen des Pufferspeichers konstant ist und der Pufferspeicher ein Fluid, insbesondere ein Gas, unter Überdruck zwischenspeichert.

Der Pufferspeicher umfasst mindestens eine Kammer in seinem Inneren, optional mehrere Kammern. Die oder jede Kammer vermag jeweils ein Fluid aufzunehmen und wieder abzugeben. Falls der Pufferspeicher mindestens zwei Kammern umfasst, so sind diese beiden Kammern bevorzugt fluiddicht voneinander getrennt und können unterschiedliche Fluide aufnehmen.

Weiterhin wird nachfolgend der Begriff "Quelle" verwendet. Im Kontext der Erfindung vermag eine Quelle ein Fluid bereitzustellen, insbesondere einen Gas-Bestandteil. Eine Quelle ist insbesondere ein stationärer Versorgungsanschluss oder eine mobile Quelle, beispielsweise ein Behälter mit dem Fluid, insbesondere eine Druckluftflasche. Möglich ist, dass mindestens zwei verschiedenartige Quellen verwendet werden, insbesondere eine stationäre Quelle und eine mobile Quelle.

Die erfindungsgemäße Versorgungs-Anordnung umfasst einen ersten Kanal mit einem Versorgungs-Verbindungselement. Eine Fluidverbindung zwischen dem Versorgungs-Verbindungselement und einer ersten Quelle ist hergestellt oder lässt sich wenigstens zeitweise herstellen. Diese erste Quelle ist eine Quelle für den ersten Gas-Bestandteil.

Die erfindungsgemäße Versorgungs-Anordnung umfasst weiterhin einen Pufferspeicher. Im Inneren des Pufferspeichers ist eine Versorgungskammer angeordnet. Zwischen der Versorgungskammer und einer zweiten Quelle ist eine Fluidverbindung hergestellt oder lässt sich dauerhaft oder wenigstens zeitweise herstellen. Diese zweite Quelle ist eine Quelle für den zweiten Gas-Bestandteil. Der zweite Gas-Bestandteil unterscheidet sich chemisch vom ersten Gas-Bestandteil. Möglich ist, dass beide Gas-Bestandteile dieselbe Komponente umfassen, beispielsweise beide Sauerstoff umfassen. Die Versorgungskammer vermag eine Menge des zweiten Gas-Bestandteils aufzunehmen und wieder abzugeben.

Weiterhin umfasst die erfindungsgemäße Versorgungs-Anordnung einen zweiten Kanal. Zwischen der Versorgungskammer und dem zweiten Kanal ist eine Versorgungs-Fluidverbindung hergestellt oder lässt sich wenigstens zeitweise herstellen.

Der erste Kanal vermag den ersten Gas-Bestandteil vom Versorgungs-Verbindungselement zu einem Mischpunkt der Versorgungs-Anordnung zu leiten. Der zweite Kanal vermag den zweiten Gas-Bestandteil vom Pufferspeicher zu diesem Mischpunkt zu leiten. Dadurch lässt sich im Mischpunkt das Gasgemisch umfassend den ersten Gas-Bestandteil und den zweiten Gas-Bestandteil erzeugen. Möglich ist auch, dass das Gasgemisch von alleine im Mischpunkt entsteht.

Die erfindungsgemäße Versorgungs-Anordnung umfasst weiterhin einen Inspirations-Kanal. Dieser Inspirations-Kanal führt vom Mischpunkt zu der patientenseitigen Koppeleinheit und vermag ein Gasgemisch, das im Mischpunkt erzeugt worden ist, vom Mischpunkt zu der patientenseitigen Koppeleinheit zu leiten.

Erfindungsgemäß münden also der erste Kanal und der zweite Kanal in den Mischpunkt, und der Inspirations-Kanal beginnt im Mischpunkt. Im Mischpunkt entsteht ein Gasgemisch, welches den ersten und den zweiten Gas-Bestandteil umfasst. Im einfachsten Fall ist der Mischpunkt ein rein mechanisches Bauteil, das diese drei Kanäle nach Art eines Y-Stücks miteinander verbindet.

Weiterhin umfasst die erfindungsgemäße Versorgungs-Anordnung eine pneumatische Steuerleitung. Diese Steuerleitung stellt eine Steuer-Fluidverbindung zwischen dem ersten Kanal und dem Pufferspeicher her. Ein Fluid kann also vom ersten Kanal durch die pneumatische Steuerleitung hindurch zum Pufferspeicher und optional umgekehrt vom Pufferspeicher durch die Steuerleitung hindurch zum ersten Kanal fließen. Der Begriff "pneumatische Steuerleitung" unterscheidet die Steuerleitung gemäß der Erfindung von einer Datenleitung und von einer elektrischen Leitung.

Der Pufferspeicher, die Versorgungs-Fluidverbindung und die Steuer-Fluidverbindung erzielen im Zusammenwirken folgenden Effekt: Zwischen
- dem Druck in der Versorgungskammer,
- dem Druck im ersten Kanal und
- dem Druck im zweiten Kanal
wird ein Druckausgleich bewirkt, und zwar auch dann, wenn der Druck flussabwärts vom Mischpunkt und daher auch der Druck im ersten Kanal und / oder der Druck im zweiten Kanal über die Zeit variieren. Bei einer künstlichen Beatmung eines Patienten variiert in der Regel in mindestens einem Kanal der jeweilige Druck.

Die Erfindung ermöglicht es, ein Gasgemisch umfassend mindestens zwei Gas-Bestandteile zu der patientenseitigen Koppeleinheit zu fördern und dadurch das Gasgemisch an oder in der patientenseitigen Koppeleinheit für die künstliche Beatmung eines Patienten bereitzustellen. Weil die Versorgungs-Anordnung dazu ausgestaltet ist, das Gasgemisch aus mindestens zwei Gas-Bestandteilen zusammenzusetzen, ermöglicht die Erfindung es in vielen Fällen, ein Gasgemisch bereitzustellen, welches auf die aktuell erforderliche künstliche Beatmung des Patienten zugeschnitten ist. Insbesondere kann reiner Sauerstoff als der zweite Gas-Bestandteil fungieren, und der Anteil von Sauerstoff in dem Gasgemisch lässt sich einstellen und bei Bedarf verändern. Als zweiter Gas-Bestandteil kann auch ein Narkosemittel fungieren.

Erfindungsgemäß ist zwischen der zweiten Quelle und dem zweiten Kanal ein Pufferspeicher angeordnet. Der zweite Gas-Bestandteil fließt bei einer Verwendung der Versorgungs-Anordnung aus der zweiten Quelle zum Pufferspeicher, in die und aus der Versorgungskammer und weiter aus dem Pufferspeicher in den zweiten Kanal. Dank des Pufferspeichers, der Steuer-Fluidverbindung und der Versorgungs-Fluidverbindung wird automatisch ein Druckausgleich zwischen dem Druck im ersten Kanal und dem Druck im zweiten Kanal bewirkt. Dadurch herrschen im ersten Kanal und im zweiten Kanal annähernd der gleiche Druck, auch wenn der Druck im ersten Kanal und / oder der Volumenfluss durch den ersten Kanal mit der Zeit variieren. Zumindest wird eine Druckdifferenz mit einem gewissen Zeitverzug automatisch abgebaut.

Diese beiden zeitlich übereinstimmenden Drücke in den beiden Kanälen erleichtern es, die beiden Gas-Bestandteile zu mischen und flussabwärts von dem Mischpunkt den Volumenfluss und / oder den Druck des Gasgemischs automatisch zu regeln. Das Regelungsziel bei dieser optionalen Regelung (closed-loop control) ist, dass der tatsächliche zeitliche Verlauf des Volumenflusses oder des Drucks im Inspirations-Kanal einem vorgegebenen zeitlichen Sollverlauf folgt. Diese Regelung wiederum erleichtert es, den Patienten künstlich zu beatmen, insbesondere bei einer unterstützenden künstlichen Beatmung, welche die eigene Atmungsaktivität des Patienten unterstützt. Die Regelung erleichtert es insbesondere, die Beatmungshübe eines Beatmungsgeräts mit der eigenen Atmungsaktivität des Patienten zu synchronisieren.

Die pneumatische Steuerleitung stellt erfindungsgemäß eine pneumatische Steuer-Fluidverbindung zwischen dem ersten Kanal und dem Pufferspeicher her. Dadurch wird ein Druckausgleich zwischen dem Druck im ersten Kanal und dem Druck im Inneren des Pufferspeichers bewirkt. Der bewirkte Druckausgleich wirkt auch auf die Versorgungskammer, bevorzugt von außen. Dank der Versorgungs-Fluidverbindung wird ein Druckausgleich zwischen dem Druck in der Versorgungskammer und dem Druck im zweiten Kanal bewirkt. Dadurch wird auch ein Druckausgleich zwischen dem Druck im ersten Kanal und dem Druck im zweiten Kanal bewirkt.

Der Pufferspeicher mit der Versorgungskammer entkoppelt pneumatisch die zweite Quelle von dem zweiten Kanal, und zwar in großem Maße unabhängig davon, mit welchem Druck und mit welchem Volumenfluss die zweite Quelle den zweiten Gas-Bestandteil bereitstellt. Die zweite Quelle kann den zweiten Gas-Bestandteil mit einem zeitlich konstanten oder zeitlich variierenden Druck und Volumenfluss bereitstellen. Daher vermeidet der Pufferspeicher die Notwendigkeit, denjenigen Druck oder Volumenfluss zu steuern oder zu regeln, mit dem die zweite Quelle den zweiten Gas-Bestandteil bereitstellt. Dies erleichtert es, eine vorhandene oder aktuell verfügbare zweite Quelle dafür zu verwenden, das Gasgemisch an der patientenseitigen Koppeleinheit bereitzustellen. Diese Wirkung ist insbesondere dann von Vorteil, wenn als zweite Quelle eine mobile oder sonstige Quelle bereitsteht, welche sich nur schwer oder überhaupt nicht regeln oder steuern lässt. Die Wirkung ist oft auch dann von Vorteil, wenn ein stationäres Versorgungsnetz verschiedene erfindungsgemäße Versorgungs-Anordnungen mit dem zweiten Gas-Bestandteil versorgt. Die mit dem Versorgungsnetz verbundenen Versorgungsanordnungen können den zweiten Gas-Bestandteil mit unterschiedlichen Drücken benötigen.

Dank der pneumatischen Steuerleitung ist es möglich, aber nicht erforderlich, den Pufferspeicher so auszugestalten, dass ein elektronisches Steuergerät den Pufferspeicher abhängig von Messwerten eines Drucksensors ansteuert. Vielmehr ist es in vielen Fällen möglich, dass der Pufferspeicher als ein rein mechanisches Bauteil ausgestaltet ist. In diesem Fall benötigt der Pufferspeicher auch keine elektrische Energie und keine Datenverbindung. In vielen Fällen ist ein solcher Pufferspeicher mechanisch stabiler und / oder robuster gegen Umgebungseinflüsse als ein elektronisch ansteuerbarer Pufferspeicher.

Möglich ist, dass ein signalverarbeitendes elektronisches Steuergerät (control unit) Messwerte von einem Drucksensor empfängt und verarbeitet, wobei dieser Drucksensor ein Maß für den Druck im ersten Kanal misst. Gemäß einer Ausgestaltung steuert das Steuergerät den Pufferspeicher abhängig von Messwerten des Drucksensors an, wobei das Ziel bei der Ansteuerung ist, dass der Druck im Inneren des Pufferspeichers, insbesondere in der Versorgungskammer, dem Druck im ersten Kanal folgt. Dank der Versorgungs-Fluidverbindung folgt dann auch der Druck im zweiten Kanal dem Druck im ersten Kanal.

Möglich ist auch, dass der oder ein Drucksensor den Druck flussabwärts vom Mischpunkt, also im Inspirations-Kanal misst. Wiederum steuert das Steuergerät gemäß einer Ausgestaltung den Pufferspeicher abhängig von den Messwerten des Drucksensors an. Das Ziel bei dieser Ansteuerung ist, dass der Druck im Inneren des Pufferspeichers dem Druck im Inspirations-Kanal folgt. Dank der Steuer-Fluidverbindung und der Versorgungs-Fluidverbindung folgen dann der Druck im ersten Kanal und der Druck im zweiten Kanal ebenfalls dem Druck im Inspirations-Kanal.

Anstelle eines Drucksensors oder zusätzlich zum Drucksensor lässt sich auch ein Sensor für den Volumenfluss durch den jeweiligen Kanal verwenden.

Erfindungsgemäß umfasst der Pufferspeicher ein Gehäuse und ein fluiddichtes Trennelement. Bevorzugt ist das Gehäuse starr. Das Trennelement ist im Inneren des Gehäuses angeordnet und bevorzugt innen am Gehäuse befestigt. Das Trennelement unterteilt das Innere des Gehäuses fluiddicht in zwei verschiedene Kammern, nämlich in die Versorgungskammer und eine Steuerkammer. Dank des Trennelements wird verhindert, dass ein Fluid von der einen Kammer in die andere Kammer gelangt. Das Trennelement ist bevorzugt so ausgestaltet, dass das Volumen der Versorgungskammer veränderlich ist, auch wenn das Gehäuse starr ist.

Möglich ist, dass die beiden Kammern zusammen das gesamte Innere des Gehäuses einnehmen. Möglich ist auch, dass ein Teil des Pufferspeichers weder zur Versorgungskammer noch zur Steuerkammer gehört. Der Begriff "fluiddicht" ist nicht notwendigerweise absolut zu verstehen, sondern kann die Möglichkeit einschließen, dass eine relativ geringe Menge eines Fluids durch unvermeidliche Undichtigkeiten hindurch von der einen Kammer in die andere Kammer gelangt.

Die Fluidverbindung zwischen dem Pufferspeicher und der zweiten Quelle verbindet die Versorgungskammer im Inneren des Gehäuses wenigstens zeitweise mit der zweiten Quelle, also mit der Quelle für den zweiten Gas-Bestandteil. Die Versorgungs-Fluidverbindung zwischen dem Pufferspeicher und dem zweiten Kanal verbindet die Versorgungskammer mit dem zweiten Kanal. Dank dieser Ausgestaltung steht die zweite Quelle in einer Fluidverbindung mit dem zweiten Kanal, wobei diese Fluidverbindung durch die Versorgungskammer hindurch führt. Die Versorgungs-Fluidverbindung bewirkt einen Druckausgleich zwischen dem Druck in der Versorgungskammer und dem Druck im zweiten Kanal. Weil das Trennelement die beiden Kammern fluiddicht voneinander trennt, wird verhindert, dass der zweite Gas-Bestandteil durch die pneumatische Steuerleitung hindurch in den ersten Kanal gelangt.

Die pneumatische Steuerleitung und daher die Steuer-Fluidverbindung verbinden die Steuerkammer im Inneren des Gehäuses mit dem ersten Kanal. Dadurch ist eine Fluidverbindung zwischen dem ersten Kanal und der Steuerkammer hergestellt, wobei diese Fluidverbindung zu der erfindungsgemäßen Steuer-Fluidverbindung gehört. Die pneumatische Steuerleitung bewirkt einen Druckausgleich zwischen dem Druck im ersten Kanal und dem Druck in der Steuerkammer.

Gemäß einer Realisierungsform dieser Ausgestaltung ist das Trennelement flexibel. Gemäß einer anderen Regierungsform ist das Trennelement starr und relativ zum Gehäuse beweglich angeordnet, beispielsweise verschiebbar oder verschwenkbar gelagert. Beispielsweise ist das Trennelement eine verschiebbar gelagerte starre Platte.

Bei beiden Realisierungsformen kann wenigstens ein Bereich des Trennelements seine Position relativ zum Gehäuse des Pufferspeichers verändern. Diese Veränderung wird insbesondere durch einen Unterschied zwischen den Drücken in den beiden Kammern verursacht und reduziert den Druckunterschied. Idealerweise baut das flexible Trennelement einen Druckunterschied zwischen den beiden Kammern vollständig ab.

Weil das Trennelement im Inneren des Gehäuses flexibel und / oder beweglich ist, stellt sich von allein ein Druckausgleich zwischen dem Druck in der Versorgungskammer und dem Druck in der Steuerkammer ein. Nachdem der Druck in einer Kammer sich verändert hat, dauert es in der Regel einige Zeit, bis auch der Druck in der anderen Kammer sich entsprechend verändert hat. Dieser Druckausgleich findet automatisch statt, ohne dass es einer Ansteuerung oder eines Eingriffs von außen bedarf. Vielmehr kann das Trennelement ein passives mechanisches Bauteil sein. Dieser Druckausgleich bewirkt über die oben beschriebene Wirkungskette einen Druckausgleich zwischen dem Druck im ersten Kanal und dem Druck im zweiten Kanal.

Erfindungsgemäß verbindet die pneumatische Steuerleitung den Pufferspeicher mit dem ersten Kanal. Dieses Merkmal lässt sich mit einer Ausgestaltung kombinieren, bei welcher der Pufferspeicher elektronisch angesteuert wird. Diese Kombination erzeugt Redundanz und ermöglicht in manchen Fällen, dass die Drücke in den beiden Kammern sich noch schneller aneinander angleichen.

Bevorzugt ist die Versorgungskammer so groß, dass sie mindestens ein Tidalvolumen zu fassen und wieder abzugeben vermag. Bekanntlich ist das Tidalvolumen das Volumen, welches ein erwachsener Patient bei einem einzigen Atemzug aufnimmt. Dank dieser Ausgestaltung vermag die Versorgungskammer bei jedem gewünschten Mischungsverhältnis eine Menge des zweiten Gas-Bestandteils bereitzustellen, die für einen einzigen Beatmungshub ausreicht.

Erfindungsgemäß ist das Trennelement als ein flexibler fluiddichter Beutel ausgestaltet oder umfasst einen flexiblen fluiddichten Beutel. Zwischen dem Gehäuse und dem Beutel tritt wenigstens bereichsweise ein Abstand auf, wodurch ein Innenraum gebildet wird. Dieser Innenraum umschließt wenigstens teilweise den Beutel. Das Gehäuse umgibt und schützt den Beutel. Das Trennelement trennt den Innenraum vom Inneren des Beutels. Das Innere des Beutels bildet eine Kammer, der Innenraum im Gehäuse um den Beutel herum eine weitere Kammer.

In einer ersten Alternative der Erfindung wird die Steuerkammer im Innenraum zwischen dem Gehäuse und dem Beutel gebildet. Die pneumatische Steuerleitung stellt die Steuer-Fluidverbindung her, welche den Innenraum zwischen dem Beutel und dem Gehäuse mit dem ersten Kanal verbindet. Die Versorgungskammer ist im Inneren des Beutels angeordnet und wird vom Beutel umschlossen. Die Versorgungs-Fluidverbindung verbindet das Innere des Beutels mit dem zweiten Kanal.

In einer zweiten Alternative der Erfindung wird die Steuerkammer im Inneren des Beutels gebildet. Die pneumatische Steuerleitung stellt die Steuer-Fluidverbindung zwischen dem Inneren des Beutels und dem ersten Kanal her. Die Versorgungskammer wird im Innenraum zwischen dem Gehäuse und dem Beutel gebildet. Die Versorgungs-Fluidverbindung verbindet diesen Innenraum mit dem zweiten Kanal.

Beide Alternativen der Erfindung ermöglichen es in vielen Fällen, einen mechanisch besonders robusten Pufferspeicher bereitzustellen. Falls der Pufferspeicher quaderförmig ist, reicht es in vielen Fällen aus, den Beutel mit einer Wand dieses Quaders zu verbinden und in dieser Wand eine Öffnung vorzusehen, durch die hindurch die Versorgungs-Fluidverbindung geführt ist.

Damit ein Druckausgleich zwischen den beiden Kanälen stattfinden kann, darf die zweite Quelle weder eine zu große Menge noch eine zu kleine Menge des zweiten Gas-Bestandteils bereitstellen. Ansonsten vermag der Pufferspeicher nicht mehr den gewünschten Druckausgleich zu bewirken. Verschiedene Ausgestaltungen sind möglich, um den Pufferspeicher zu überwachen und bei Bedarf eine automatische Änderung oder wenigstens einen Alarm auszulösen.

In einer Ausgestaltung ist das Gehäuse durchsichtig, oder ein Sichtfenster ist in das Gehäuse eingelassen. Ein Benutzer kann von außen visuell den Pufferspeicher und insbesondere die Position des Trennelements überwachen. Insbesondere lässt sich die unerwünschte Situation feststellen, dass die tatsächliche Position des Trennelements stark von einer Position, die sich bei einem Druckausgleich zwischen den beiden Kammern einstellt, abweicht. Falls das Trennelement einen Beutel umfasst, so lassen sich visuell sowohl das unerwünschte Ereignis, dass der Beutel stark zusammengedrückt ist (zu kleiner Druck im Beutel / zu großer Druck im Innenraum zwischen den Beutel und dem Gehäuse), als auch das unerwünschte Ereignis, dass der Beutel gegen die Innenwand des Gehäuses gedrückt ist (zu großer Druck im Beutel / zu kleiner Druck im Innenraum), feststellen.

Eine andere Realisierungsform erspart die Notwendigkeit, den Pufferspeicher visuell zu überwachen. In einer Realisierungsform der Ausgestaltung mit den beiden Kammern im Inneren des Pufferspeichers umfasst die Versorgungs-Anordnung eine Sensor-Anordnung. In einer Realisierungsform vermag diese Sensor-Anordnung ein Maß für eine Druck-Differenz zu messen, nämlich für die Differenz zwischen dem Druck in der Versorgungskammer und dem Druck in der Steuerkammer. Falls die gemessene Druck-Differenz für eine ausreichend lange Zeitspanne außerhalb eines vorgegebenen Bereichs um den Nullpunkt liegt, so wird eine Meldung generiert und bevorzugt in einer von einem Menschen wahrnehmbaren Form ausgegeben. In einer anderen Realisierungsform vermag die Sensor-Anordnung ein Maß für das Volumen des Beutels zu messen. Beispielsweise detektiert die Sensor-Anordnung das unerwünschte Ereignis, dass der Beutel von innen gegen das Gehäuse gedrückt wird, was auf ein zu großes Volumen des Beutels hindeutet.

Die Realisierungsform mit dem durchsichtigen Gehäuse oder dem Sichtfenster lässt sich mit der Realisierungsform umfassend die Sensor-Anordnung kombinieren.

Eine mögliche Abhilfe ist, dass wenigstens zeitweise der Druck verändert wird, mit dem die zweite Quelle den zweiten Gas-Bestandteil bereitzustellen vermag.

Erfindungsgemäß vermag die Versorgungs-Anordnung ein Gasgemisch umfassend einen ersten Gas-Bestandteil und einen zweiten Gas-Bestandteil zu erzeugen. Der erste Gas-Bestandteil wird von der ersten Quelle bereitgestellt, der zweite Gas-Bestandteil von der zweiten Quelle. In einer Ausgestaltung vermag die Versorgungs-Anordnung ein Gasgemisch zu erzeugen, das zusätzlich einen dritten Gas-Bestandteil umfasst, beispielsweise ein Narkosemittel. Eine dritte Quelle stellt den dritten Gas-Bestandteil bereit.

In einer anderen Ausgestaltung vermögen sowohl die zweite Quelle als auch eine weitere Quelle den zweiten Gas-Bestandteil bereitzustellen. Dadurch wird Redundanz hergestellt. Die weitere Quelle stellt den zweiten Gas-Bestandteil bevorzugt mit einem höheren Druck als die zweite Quelle bereit. Beispielsweise ist die zweite Quelle eine mobile Quelle, insbesondere ein Sauerstoffgenerator, und die weitere Quelle ist ein stationärer Versorgungsanschluss oder umfasst eine unter Druck stehende Flasche. In einer Alternative vermag die weitere Quelle einen dritten Gas-Bestandteil bereitzustellen.

Die Versorgungs-Anordnung umfasst bei dieser Anwendung bevorzugt zusätzlich einen pneumatischen Druckminderer mit einem Vordruck-Eingang und einem Hinterdruck-Ausgang. Außerdem umfasst die Versorgungs-Anordnung einen dritten Kanal für den zweiten oder den dritten Gas-Bestandteil. In einer Alternative führt dieser dritte Kanal zu demjenigen Mischpunkt, zu dem auch der erste Kanal und der zweite Kanal führen. In einer anderen Alternative führt der dritte Kanal zu einem weiteren Mischpunkt, der in einer Fluidverbindung mit dem Mischpunkt steht und flussaufwärts oder flussabwärts von dem Mischpunkt angeordnet sein kann, in dem der erste und der zweite Gas-Bestandteil miteinander vermischt werden. Der Inspirations-Kanal führt vom Mischpunkt oder vom weiteren Mischpunkt zur patientenseitigen Koppeleinheit.

Zwischen dem Vordruck-Eingang des Druckminderers und der weiteren Quelle ist eine Fluidverbindung hergestellt oder lässt sich herstellen, sodass der zweite oder der dritte Gas-Bestandteil von der weiteren Quelle in den Druckminderer eingespeist werden kann. Der Hinterdruck-Ausgang des Druckminderers ist mit dem dritten Kanal verbunden, sodass der Druckminderer den zweiten oder den dritten Gas-Bestandteil in den dritten Kanal einspeisen kann.

Der Druckminderer stellt den zweiten oder den dritten Gas-Bestandteil an seinem Hinterdruck-Ausgang bereit. Zumindest in der Regel ist der Druck am Hinterdruck-Ausgang kleiner als der Druck am Vordruck-Eingang, d.h. der Druckminderer reduziert den Druck. Der Druckminderer ist so ausgestaltet, dass diese Bereitstellung am Hinterdruck-Ausgang die folgende Wirkung hat: Der zeitliche Verlauf des Drucks am Hinterdruck-Ausgang folgt dem zeitlichen Verlauf des Drucks am Versorgungs-Verbindungselement.

Möglich ist, dass ein Drucksensor den Druck im ersten Kanal misst und ein signalverarbeitendes Steuergerät (control unit) den Druckminderer ansteuert, und zwar abhängig von einem Signal des Drucksensors für den ersten Kanal.

Möglich ist, dass ein signalverarbeitendes Steuergerät den Druckminderer elektronisch ansteuert, so dass der Druck, mit dem der Druckminderer am Hinterdruck-Ausgang den zweiten Gas-Bestandteil bereitstellt, dem Druck im ersten Kanal folgt.

In einer anderen Realisierungsform der Ausgestaltung mit dem Druckminderer umfasst der Druckminderer zusätzlich einen Steuerdruck-Eingang. Die Versorgungs-Anordnung umfasst eine weitere pneumatische Steuerleitung. Die weitere pneumatische Steuerleitung stellt eine weitere pneumatische Steuer-Fluidverbindung zwischen dem ersten Kanal und dem Steuerdruck-Eingang her. Die weitere pneumatische Steuerleitung erspart die Notwendigkeit, den Druckminderer elektronisch anzusteuern. Vielmehr ist es möglich, den Druckminderer als ein rein mechanisches und pneumatisches Bauteil auszugestalten. Die beiden Realisierungsformen mit der elektronischen Ansteuerung und der weiteren pneumatischen Steuerleitung lassen sich auch kombinieren, was Redundanz bewirkt.

Die Erfindung betrifft weiterhin ein System, welches eine patientenseitige Koppeleinheit mit einem Gasgemisch zu versorgen vermag. Dieses Gasgemisch umfasst einen ersten Gas-Bestandteil und einen zweiten Gas-Bestandteil. Das Versorgungs-System umfasst eine erste Quelle, eine zweite Quelle und eine erfindungsgemäße Versorgungs-Anordnung. Die erste Quelle stellt den ersten Gas-Bestandteil bereit, die zweite Quelle den zweiten Gas-Bestandteil. Zwischen dem Versorgungs-Verbindungselement des ersten Kanals der Versorgungs-Anordnung und der ersten Quelle ist wenigstens zeitweise eine Fluidverbindung hergestellt. Zwischen der Versorgungskammer im Pufferspeicher der Versorgungs-Anordnung und der zweiten Quelle ist ebenfalls wenigstens zeitweise eine Fluidverbindung hergestellt.

Die gerade beschriebenen Vorteile der erfindungsgemäßen Versorgungs-Anordnung gelten auch für dieses Versorgungs-System. Mögliche vorteilhafte Ausgestaltungen der erfindungsgemäßen Versorgungs-Anordnung sind auch Vorteile des Versorgungs-Systems.

In einer Ausgestaltung umfasst das Versorgungs-System zusätzlich eine weitere Quelle, die ebenfalls den zweiten Gas-Bestandteil bereitzustellen vermag, und zwar bevorzugt mit einem höheren Druck als die zweite Quelle, oder auch einen dritten Gas-Bestandteil. Die Versorgungs-Anordnung umfasst zusätzlich einen Druckminderer. Ein Vordruck-Eingang des Druckminderers steht in einer Fluidverbindung mit der weiteren Quelle, und ein Hinterdruck-Ausgang des Druckminderers steht in einer Fluidverbindung mit dem zweiten Kanal. Der Druck am Hinterdruck-Ausgang folgt dem Druck im ersten Kanal.

Weiterhin betrifft die Erfindung ein Beatmungs-System, welches einen Patienten künstlich zu beatmen vermag. Dieses Beatmungs-System umfasst die oben bereits erwähnte patientenseitige Koppeleinheit, eine Fluidfördereinheit und eine erfindungsgemäße Versorgungs-Anordnung oder ein erfindungsgemäßes Versorgungs-System. Die Fluidfördereinheit vermag ein Gasgemisch zu der patientenseitigen Koppeleinheit zu fördern, wobei dieses Gasgemisch von der Versorgungs-Anordnung oder von dem Versorgungs-System erzeugt worden ist.

Die Fluidfördereinheit kann flussaufwärts oder flussabwärts vom Mischpunkt angeordnet sein.

Im Folgenden wird die Erfindung anhand eines Ausführungsbeispiels beschrieben. Hierbei zeigt
- Figur 1: schematisch, wie eine patientenseitige Koppeleinheit mit einem Gasgemisch aus Atemluft und reinem Sauerstoff versorgt wird, wobei zwei verschiedene Quellen den reinen Sauerstoff mit unterschiedlichen Drücken bereitstellen;
- Figur 2: schematisch eine Abwandlung der Ausgestaltung von Figur 1, wobei eine einzige Quelle den reinen Sauerstoff bereitstellt;
- Figur 3: einen beispielhaften zeitlichen Verlauf des Volumenflusses und des Drucks im Inspirations-Kanal;
- Figur 4: eine beispielhafte Kopplung eines Pufferspeichers mit einem Beutel an das Beatmungsgerät;
- Figur 5: eine andere beispielhafte Kopplung eines Pufferspeichers mit einem Beutel an das Beatmungsgerät;
- Figur 6: eine beispielhafte Kopplung eines Pufferspeichers mit einer Membrane an das Beatmungsgerät.

Im Ausführungsbeispiel wird die Erfindung benutzt, um einen Patienten Pt künstlich zu beatmen. Am oder im Körper des Patienten Pt ist eine patientenseitige Koppeleinheit 9 befestigt, beispielsweise eine Atemmaske oder ein Tubus oder ein Katheter.

Ein nur schematisch gezeigtes Beatmungsgerät 100 führt eine Abfolge von Beatmungshüben aus und fördert in jedem Beatmungshub ein Gasgemisch zu der patientenseitigen Koppeleinheit 9 und damit zu dem Patienten Pt. Die erfindungsgemäße Versorgungs-Anordnung gehört zum Beatmungsgerät 100. Das Gasgemisch enthält einen Anteil (Vol-%) von Sauerstoff, wobei ein Benutzer einen Sollwert für diesen Anteil vorgegeben hat. Dieser Sauerstoff-Anteil kann oberhalb des Anteils von Sauerstoff in der Atemluft liegen. Um den Anteil von Sauerstoff gegenüber der Atemluft zu vergrößern, wird im Ausführungsbeispiel ein Gasgemisch aus Atemluft und reinem Sauerstoff erzeugt. Das Gasgemisch kann zusätzlich ein Narkosemittel enthalten, sodass der Patient Pt sediert oder narkotisiert wird.

Ein Benutzer gibt einen gewünschten Sauerstoff-Anteil im Gasgemisch vor. Beispielsweise stellt der Benutzer manuell den Sauerstoffgehalt an einem Drehknopf 30 ein.

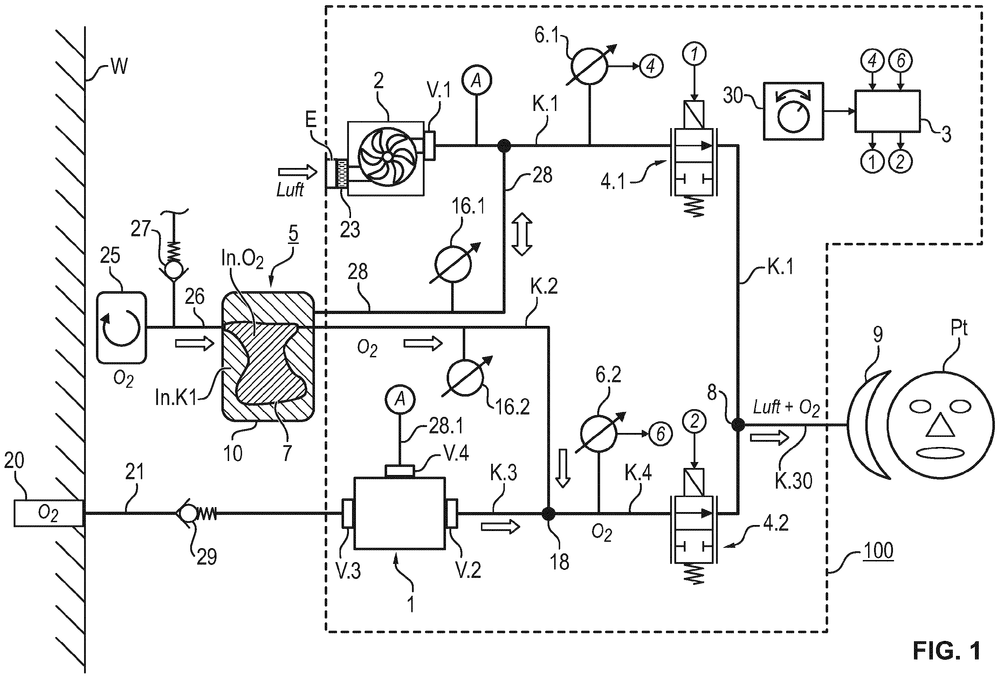

Figur 1 zeigt eine erste Ausgestaltung einer Anordnung, um dieses Gasgemisch mit einem höheren Sauerstoffanteil zu erzeugen und zur patientenseitigen Koppeleinheit 9 zu fördern. Ein erster Kanal K.1 stellt die Atemluft bereit. Ein zweiter Kanal K.2 und ein dritter Kanal K.3 stellen reinen Sauerstoff bereit. Der zweite Kanal K.2 und der dritte Kanal K.3 münden in einen Mischpunkt 18. Im Mischpunkt 18 beginnt ein vierter Kanal K.4, der ebenfalls reinen Sauerstoff leitet. Der Begriff "Kanal" bezeichnet eine Fluidfördereinheit, welche ein Fluid entlang einer Trajektorie zu leiten vermag.

Der erste Kanal K.1 und der vierte Kanal K.4 münden in einen Mischpunkt 8. Von diesem Mischpunkt 8 führt ein Inspirations-Kanal K.30, beispielsweise ein Schlauch für die Einatmung und optional ein Zwei-Lumen-Schlauch mit einem zusätzlichen Schlauch für die Ausatmung, zur patientenseitigen Koppeleinheit 9. Dieser Inspirations-Kanal K.30 leitet das Gemisch aus Luft und reinem Sauerstoff zu der patientenseitigen Koppeleinheit 9.

Möglich ist auch, dass das Gasgemisch einen dritten Gas-Bestandteil enthält, beispielsweise ein Narkosemittel. Bei dieser Ausgestaltung leitet ein dritter Kanal (nicht gezeigt) den dritten Gas-Bestandteil zum Mischpunkt 8.

Ein Gebläse 2 oder Pumpe oder eine sonstige Fluidförderereinheit des Beatmungsgeräts 100 saugt Umgebungsluft durch einen Einlass E der Förderereinheit 2 an und speist die angesaugte Luft in den ersten Kanal K.1 ein.

Zwischen dem Einlass E und dem Gebläse 2 ist ein Filter 23 angeordnet. In der Anwendung gemäß Figur 1 fungiert der Einlass E als die Quelle für den ersten Gas-Bestandteil.

Ein Versorgungs-Verbindungselement V.1 des ersten Kanals K.1 ist mit einem Versorgungs-Ausgang des Gebläses 2 verbunden. Der Druck im ersten Kanal K.1 folgt idealerweise einem vorgegebenen zeitlichen Verlauf, ist beispielsweise zeitlich konstant. Der Druck im ersten Kanal K.1 liegt bevorzugt stets oberhalb des maximalen Beatmungsdrucks, also oberhalb des maximalen Drucks, mit dem das Gasgemisch zur patientenseitigen Koppeleinheit 9 und weiter in die Lunge des Patienten Pt gefördert wird, und liegt bevorzugt zwischen 20 mbar und 100 mbar.

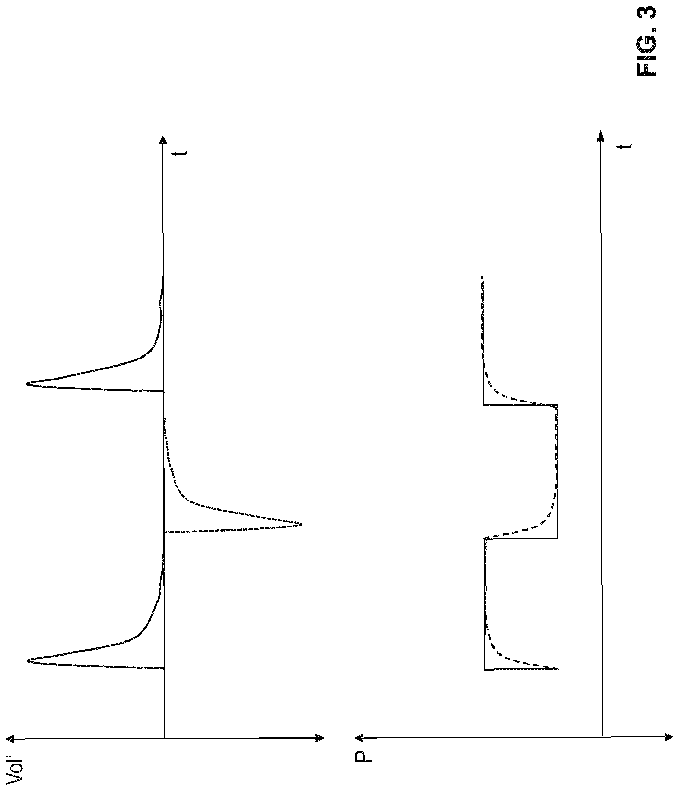

Der Volumenfluss, also der Fluss von Gas pro Zeiteinheit, durch den Inspirations-Kanal K.30 zur patientenseitigen Koppeleinheit 9 soll einem vorgegebenen zeitlichen Verlauf folgen. Figur 3 zeigt oben einen beispielhaften geforderten zeitlichen Verlauf des Volumenflusses (Vol') und unten einen beispielhaften geforderten zeitlichen Verlauf des Drucks (P). Werte oberhalb der x-Achse bedeuten einen Fluss des Gasgemischs zum Patienten Pt hin (Einatmung), Werte unterhalb einen Fluss vom Patienten Pt weg (Ausatmung).

Ein signalverarbeitendes Steuergerät 3 führt eine Regelung (closed-loop control) durch, wobei der tatsächliche Volumenfluss Vol' die Regelgröße und der vorgegebene zeitliche Verlauf des Volumenflusses die Führungsgröße ist. Der tatsächliche Volumenfluss Vol' zur patientenseitigen Koppeleinheit 9 ist die Summe der Volumenflüsse durch die beiden Kanäle K.1 und K.4, die in den Mischpunkt 8 münden.

Ein Volumenfluss-Sensor 6.1 misst ein Maß für den tatsächlichen Volumenfluss im ersten Kanal K.1. Beispielsweise misst der Volumenfluss-Sensor 6.1 eine Druck-Differenz zwischen zwei in Fließrichtung voneinander beabstandeten Messpunkten im ersten Kanal K.1. Das Steuergerät 3 steuert ein Proportionalventil 4.1 an und verändert dadurch bei Bedarf den Volumenfluss durch den ersten Kanal K.1 flussabwärts vom Proportionalventil 4.1 zum Mischpunkt 8.

Im vierten Kanal K.4 sind ein Volumenfluss-Sensor 6.2 und ein Proportionalventil 4.2 angeordnet. Diese Bauteile funktionieren so wie die entsprechenden Bauteile im ersten Kanal K.1. Das Steuergerät 3 steuert das Proportionalventil 4.2 mit dem Regelungsziel an, dass der tatsächliche Volumenfluss durch den vierten Kanal K.4 einem vorgegebenen zeitlichen Verlauf folgt.

Wie bereits dargelegt, wird ein Gasgemisch vom Mischpunkt 8 zur patientenseitigen Koppeleinheit 9 gefördert. Der tatsächliche Volumenfluss Vol' dieses Gasgemischs flussabwärts vom Mischpunkt 8 soll einem vorgegebenen zeitlichen Verlauf folgen. Außerdem ist ein Anteil von Sauerstoff im Gasgemisch vorgegeben, bevorzugt als Vol-% vorgegeben. Dieser Sauerstoffanteil kann zeitlich konstant oder zeitlich variabel sein. Die beiden Proportionalventile 4.1 und 4.2 können jeweils einen Volumenfluss verändern, nicht aber einen Sauerstoff-Anteil.

Durch den vierten Kanal K.4 fließt reiner Sauerstoff, durch den ersten Kanal K.1 Atemluft. Aus dem geforderten Anteil von Sauerstoff im Gasgemisch sowie dem bekannten Anteil von Sauerstoff in Luft resultiert ein gefordertes Soll-Verhältnis zwischen den beiden Volumenflüssen durch die beiden Kanäle K.1 und K.4. Aus dem vorgegebenen zeitlichen Verlauf des Volumenflusses zur patientenseitigen Koppeleinheit 9 und dem Soll-Verhältnis der Volumenflüsse resultieren ein Soll-Verlauf des Volumenflusses im ersten Kanal K.1 und ein Soll-Verlauf des Volumenflusses im vierten Kanal K.4. Das Steuergerät 3 oder ein übergeordnetes Steuergerät berechnen diese beiden Soll-Verläufe für die beiden Kanäle K.1 und K.4, und das Steuergerät 3 steuert die beiden Proportionalventile 4.1 und 4.2 abhängig von diesen beiden Soll-Verläufen an. Das Steuergerät 3 führt also zwei Regelungen des Volumenflusses durch, nämlich eine im ersten Kanal K.1 und eine im vierten Kanal K.4.

Der zweite Kanal K.2 liefert reinen Sauerstoff mit einem niedrigeren Druck als der dritte Kanal K.3. Im gezeigten Ausführungsbeispiel ist der zweite Kanal K.2 über eine Verbindungsleitung 26 mit einer schematisch gezeigten und bevorzugt mobilen zweiten Quelle 25 verbunden. Ein Überdruckventil 27 öffnet sich, wenn der Druck in der Verbindungsleitung 26 oberhalb einer vorgegebenen Schranke von beispielsweise 100 mbar liegt, und begrenzt dadurch den Druck in der Verbindungsleitung 26.

In einer Ausgestaltung erhält die zweite Quelle 25 aus der Umgebung Atemluft und absorbiert durch mehrfache Druckwechsel aus der Atemluft einen größeren Teil des Stickstoffs. Hierbei wird die Umgebungsluft unter erhöhtem Druck in einen ersten Tank mit Zeolithen eingespeichert. Dort wird ein Teil des Stickstoffs absorbiert. Das komprimierte Gas mit einem verringerten Stickstoffanteil wird in einen nachfolgenden zweiten Tank geleitet. In dem ersten Tank sinkt der Druck, die Luft expandiert, der Stickstoff wird desabsorbiert und als Abfallgas ausgespült. Dieser Prozess wird mehrfach durchlaufen, bis eine ausreichend hohe Sauerstoffkonzentration erreicht ist. Eine auf diese Weise arbeitende Quelle kann ein Gasgemisch mit maximal 95 Vol-% Sauerstoff liefern. Edelgase in der Umgebungsluft verbleiben in diesem Gasgemisch. Bei der Berechnung der Soll-Volumenflüsse wird berücksichtigt, welchen Sauerstoffanteil das Gasgemisch von der zweiten Quelle aufweist.

Möglich ist auch, dass die mobile Quelle 25 chemische Stoffe enthält, beispielsweise schüttfähige oder feste Stoffe, die als Reaktion auf eine Aktivierung eine exothermisch chemische Reaktion durchlaufen, beispielsweise bei Kontakt mit Feuchtigkeit. Bei dieser Reaktion liefert die Quelle 25 Sauerstoff. Der Stoff ist beispielsweise Natriumchlorat. Die Quelle umfasst beispielsweise mindestens eine Chloratkerze. Beispielsweise läuft in der mobilen Quelle 25 die chemische Reaktion 2 NaClO₃ → 2 NaCl + 3 O₂ ab. In der Regel vermag eine solche mobile Quelle 25 Sauerstoff mit einem Druck von höchstens 500 mbar bereitzustellen.

Der dritte Kanal K.3 erhält reinen Sauerstoff (O2) aus einer Versorgungsleitung 21, die in Verbindung mit einem Versorgungsanschluss 20 steht. Im gezeigten Beispiel ist dieser Versorgungsanschluss 20 stationär in einer Wand W angeordnet und wird aus einer stationären Krankenhaus-Infrastruktur versorgt. Möglich ist auch, dass der dritte Kanal K.3 reinen Sauerstoff aus unter Druck stehenden Flaschen erhält. Der Versorgungsanschluss 20 stellt den reinen Sauerstoff bevorzugt mit einem Druck bereit, der zwischen 2 bar und 8 bar liegt.

Ein optionales Rückschlagventil 29 in der Versorgungsleitung 21 verhindert, dass reiner Sauerstoff zurück in den Versorgungsanschluss 20 und in die Krankenhaus-Infrastruktur gepresst wird.

Ein pneumatischer Druckminderer 1 umfasst einen Vordruck-Eingang V.3 und einen Hinterdruck-Ausgang V.2. Der Vordruck-Eingang V.3 ist mit der Versorgungsleitung 21 verbunden, der Hinterdruck-Ausgang V.2 mit dem zweiten Kanal K.2. Der Druckminderer 1 reduziert den Druck des reinen Sauerstoffs, den der Versorgungsanschluss 20 bereitstellt. Bevorzugt umfasst der pneumatische Druckminderer 1 zusätzlich einen Steuerdruck-Eingang V.4.

Im gezeigten Ausführungsbeispiel stellen also zwei Quellen unabhängig voneinander reinen Sauerstoff bereit, nämlich der Versorgungsanschluss 20 und die bevorzugt mobile Quelle 25. Dank dieser Redundanz steht auch dann noch reiner Sauerstoff zur Verfügung, wenn eine der beiden Quellen 20, 25 ausgefallen oder abgeschaltet ist. Im gezeigten Ausführungsbeispiel sind die beiden Quellen 20, 25 außerhalb des Beatmungsgeräts 100 angeordnet.

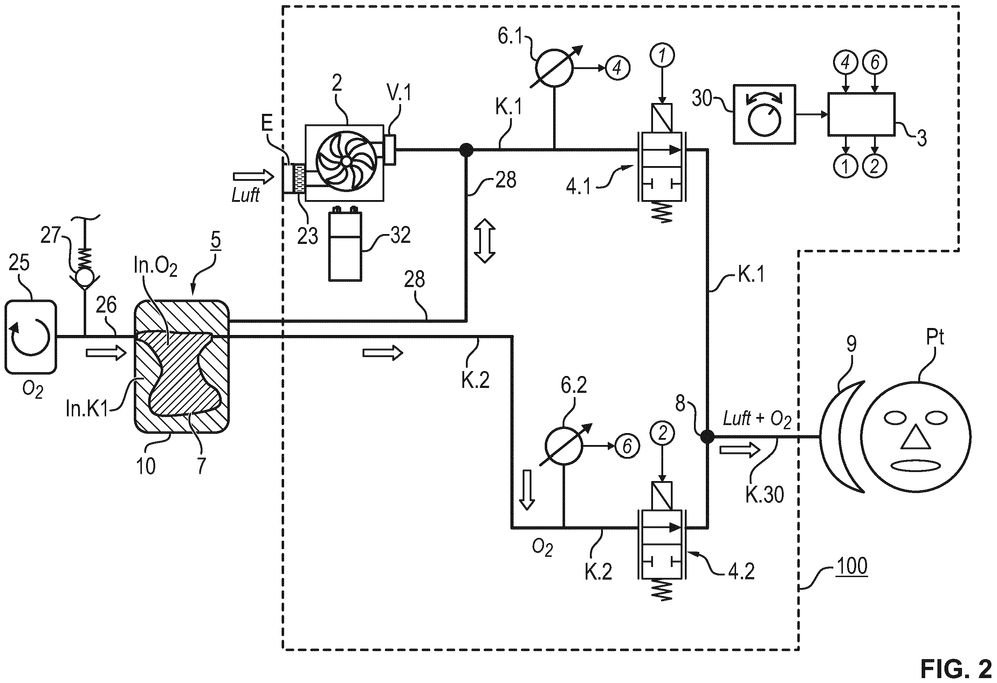

Figur 2 zeigt schematisch eine Abwandlung des Beatmungsgeräts 100 von Figur 1. Gleiche Bezugszeichen haben die gleiche Bedeutung wie in Figur 1.

Bei dieser Abwandlung besitzt das Beatmungsgerät 100 eine eigene Spannungsversorgungseinheit 32, beispielsweise eine Menge von wiederaufladbaren Batterien (Akkumulatoren), wobei die Spannungsversorgungseinheit 32 insbesondere das Gebläse 2 antreibt und die elektrische Energie für das Steuergerät 3, die Sensoren 6.1, 6.2 und die Proportionalventile 4.1, 4.2 bereitstellt. Das Beatmungsgerät 100 ist daher unabhängig von einem stationären Spannungsversorgungsnetz und wird beispielsweise für eine Notfallversorgung eines Patienten Pt verwendet, insbesondere an Bord eines Fahrzeugs oder Flugzeugs oder an einem Unfallort. In dieser Ausgestaltung steht kein stationärer Versorgungsanschluss 20 zur Verfügung. Daher werden auch keine Versorgungsleitung 21 und kein Druckminderer 1 und kein dritter Kanal K.3 und kein vierter Kanal K.4 benötigt. Der zweite Kanal K.2 führt vom Pufferspeicher 5 zum Mischpunkt 8. Der reine Sauerstoff wird ausschließlich von der Quelle 25 bereitgestellt.

Soweit nicht anders erwähnt, bezieht sich die nachfolgende Beschreibung sowohl auf die Ausgestaltung gemäß Figur 1 als auch auf die Ausgestaltung gemäß Figur 2.

Der Druck im ersten Kanal K.1 kann zeitlich variieren, auch in dem Abschnitt zwischen dem Versorgungs-Verbindungselement V.1 und dem Proportionalventil 4.1. Im Ausführungsbeispiel sollen sowohl der Druck im zweiten Kanal K.2 als auch bei der Ausgestaltung gemäß Figur 1 der Druck im dritten Kanal K.3 dem Druck im ersten Kanal K.1 folgen, idealerweise ohne Zeitverzug. Der stationäre Versorgungsanschluss 20 stellt reinen Sauerstoff mit einem Druck bereit, der idealerweise zeitlich konstant ist. Die bevorzugt mobile Quelle 25 stellt ebenfalls reinen Sauerstoff bereit, aber mit einem Druck, der zeitlich veränderlich sein kann, wobei der zeitliche Verlauf des Drucks in der Verbindungsleitung 26 in der Regel nicht mit dem Druck im ersten Kanal K.1 synchronisiert ist und z.B. von Vorgängen in der Quelle 25 abhängt. Die Erfindung erspart die Notwendigkeit, die mobile Quelle 25 mit dem Druck im ersten Kanal K.1 oder mit den Beatmungshüben zu synchronisieren.

Im Folgenden wird zunächst beschrieben, wie erreicht wird, dass der zeitliche Verlauf des Drucks im zweiten Kanal K.2 dem zeitlichen Verlauf des Drucks im ersten Kanal K.1 folgt. Wie in Figur 1 und Figur 2 zu sehen ist, ist zwischen dem Ausgang der Verbindungsleitung 26 und dem Eingang des zweiten Kanals K.2 ein Pufferspeicher 5 angeordnet. Die Begriffe "Eingang" und "Ausgang" beziehen sich auf die Fließrichtung des Sauerstoffs zum Mischpunkt 18 bzw. 8. Im Ausführungsbeispiel ist dieser Pufferspeicher 5 außerhalb des Beatmungsgeräts 100 angeordnet und lässt sich lösbar mit dem Beatmungsgerät 100 verbinden. Er kann stattdessen auch ein Bestandteil des Beatmungsgeräts 100 sein.

Der Pufferspeicher 5 umfasst ein starres Gehäuse 10 und ein flexibles fluiddichtes Trennelement 7, 7.1, welches vollständig im Inneren des Gehäuses 10 angeordnet ist. Das Trennelement 7, 7.1 unterteilt das Innere des Gehäuses 10 fluiddicht in zwei Kammern, nämlich eine Versorgungskammer In.O2 und eine Steuerkammer In.K1. Beide Kammern In.O2, In.K1 sind fluiddicht von der Umgebung abgedichtet, wozu das Gehäuse 10 beiträgt.

Die Verbindungsleitung 26 ist durch eine eingangsseitige Öffnung im Gehäuse 10 hindurch geführt, der zweite Kanal K.2 durch eine ausgangsseitige Öffnung im Gehäuse 10. Die Versorgungskammer In.O2 steht eingangsseitig in einer Fluidverbindung mit der Verbindungsleitung 26 und ausgangsseitig in einer Versorgungs-Fluidverbindung mit dem zweiten Kanal K.2. Bevorzugt sind die Verbindungsleitung 26 und der zweite Kanal K.2 fluiddicht mit dem Gehäuse 10 verbunden oder verbindbar. In einer Ausgestaltung wird die Versorgungs-Fluidverbindung durch eine Öffnung im Gehäuse 10 hergestellt, wobei der zweite Kanal K.2 fluiddicht mit dieser Öffnung verbunden ist.

Eine pneumatische Steuerleitung 28 ist mit dem Gehäuse 10 mechanisch verbunden und stellt eine Steuer-Fluidverbindung zwischen demjenigen Abschnitt des ersten Kanals K.1, der vom Versorgungs-Verbindungselement V.1 zum Proportionalventil 4.1 führt, und der Steuerkammer In.K1 her.

Dank der pneumatischen Steuerleitung 28 folgt der Druck in der Steuerkammer In.K1 dem zeitlich veränderlichen Druck im ersten Kanal K.1. Weil das Trennelement 7, 7.1 fluiddicht und flexibel ist, folgt der Druck in der Versorgungskammer In.O2 dem zeitlich veränderlichen Druck in der Steuerkammer In.K1, zumindest solange die Drücke in den beiden Kammern In.O2, In.K1 sich nicht stärker als eine bauartbedingte Schranke voneinander unterscheiden. Umgekehrt kann der Druck in der Versorgungskammer In.O2 über den Innenraum In.K1 und die Steuerleitung 28 auf den Druck im ersten Kanal K.1 rückwirken. Idealerweise haben der Druck im ersten Kanal K.1, der Druck in der pneumatische Steuerleitung 28, in der Steuerkammer In.K1, der Druck in der Versorgungskammer In.O2 und der Druck im zweiten Kanal K.2 den gleichen zeitlichen Verlauf. In der Praxis treten unvermeidliche Verzögerungen ein, u.a. weil unvermeidliche Undichtigkeiten und manchmal auch Turbulenzen auftreten. Außerdem haben zwangsläufig die beiden Kammern In.O2, In.K1 im Gehäuse 10 nur jeweils ein bestimmtes Volumen und können Druck-Differenzen nur bis zu einem bestimmten Grad ausgleichen.

In der Ausgestaltung gemäß Figur 1 stellt eine weitere pneumatische Steuerleitung 28.1 eine weitere Steuer-Fluidverbindung zwischen dem ersten Kanal K.1 und dem Steuerdruck-Eingang des Druckminderers 1 her. In einer ersten Realisierungsform ist diese weitere pneumatische Steuerleitung 28.1 direkt mit dem ersten Kanal K.1 verbunden, in einer zweiten Realisierungsform mit der pneumatischen Steuerleitung 28. Dank dieser weiteren pneumatischen Steuerleitung 28.1 folgt der Druck am Hinterdruck-Ausgang V.2 des Druckminderers 1 dem Druck im ersten Kanal K.1. Dank der weiteren pneumatischen Steuerleitung 28.1 ist es nicht erforderlich, dass das Steuergerät 3 den Druckminderer 1 oder die Quelle 20 ansteuert. Vielmehr kann der Druckminderer 1 als ein rein pneumatisches und mechanisches Bauteil ausgestaltet sein.

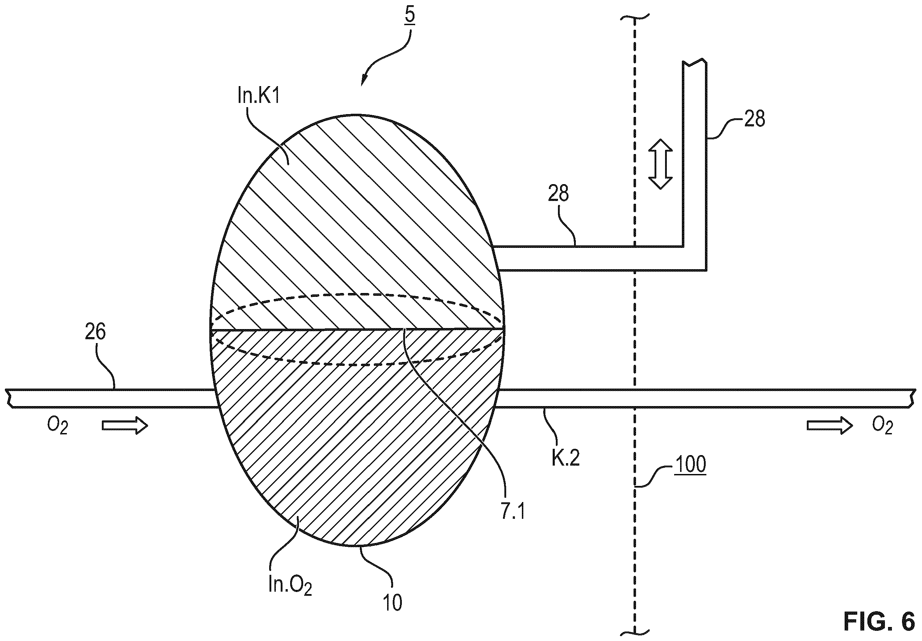

In der Realisierungsform, die in Figur 1, Figur 2, Figur 4 und Figur 5 gezeigt wird, ist dieses Trennelement 7, 7.1 als ein flexibler Beutel 7 ausgestaltet. In einer anderen Realisierungsform ist das Trennelement 7, 7.1 als eine Membrane 7.1 ausgestaltet, vgl. Figur 6. Die nachfolgende Beschreibung bezieht sich auf einen Beutel 7 als dem Trennelement. Bei der beschriebenen Realisierungsform wird die Versorgungskammer In.O2 im Inneren des Beutels 7 gebildet und die Steuerkammer In.K1 in demjenigen Bereich im Inneren des Gehäuses 10 und außerhalb des Beutels 7. Die Steuerkammer In.K1 umgibt bevorzugt vollständig den Beutel 7. Der Beutel 7 verhindert, dass eine Fluidverbindung zwischen der Quelle 25 und dem ersten Kanal K.1 auftritt.

Falls der Druck, mit dem die zweite Quelle 25 reinen Sauerstoff in die Verbindungsleitung 26 einspeist, aktuell größer als der Druck im Beutel 7 ist, so wird der Beutel 7 gedehnt und dadurch gegen den Druck in der Steuerkammer In.K1 gedehnt. Umgekehrt drückt der Druck in der Steuerkammer In.K1 den Beutel 7 zusammen, falls der Druck der Quelle 25 aktuell kleiner als der Druck in der Steuerkammer In.K1 ist.

Zwei unerwünschte Ereignisse können auftreten, sollten aber so weit als möglich vermieden werden:
- Die Quelle 25 führt dem Beutel 7 zu wenig reinen Sauerstoff zu (zu geringer Volumenfluss). Als Folge hiervon drückt der Druck in der Steuerkammer In.K1 den Beutel 7 völlig zusammen.
- Die Quelle 25 führt den Beutel 7 zu viel reinen Sauerstoff zu (zu großer Volumenfluss). Als Folge hiervon wird der Beutel 7 gegen den Druck in der Steuerkammer In.K1 stark gedehnt und wird von innen gegen das Gehäuse 10 gepresst.

In einer Realisierungsform ist das Gehäuse 10 durchsichtig, oder in das Gehäuse 10 ist ein Fenster eingelassen. Ein Benutzer kann von außen visuell den Beutel 7 inspizieren und insbesondere feststellen, ob der Beutel 7 völlig zusammengedrückt oder aber von innen gegen das Gehäuse 10 gepresst ist.

In einer anderen Realisierungsform vermag ein Sensor, beispielsweise ein Kontaktschalter, das unerwünschte Ereignis zu detektieren und zu melden, dass der Beutel 7 von innen gegen das Gehäuse 10 gepresst ist. Außerdem erzeugt in einer Ausgestaltung der Volumenfluss-Sensor 6.2 eine Meldung, wenn der Volumenfluss durch den zweiten Kanal K.2 zu gering ist. Ein solcher zu geringer Volumenfluss kann dadurch verursacht werden, dass die Quelle 25 zu wenig reinen Sauerstoff bereitstellt.

In einer weiteren Realisierungsform misst ein Drucksensor 16.1 den Druck in der pneumatischen Steuerleitung 28. Dieser Druck stimmt idealerweise mit dem Druck in der Steuerkammer In.K1 über ein. Ein weiterer Drucksensor 16.2 misst den Druck in dem zweiten Kanal K.2. Dieser Druck stimmt idealerweise mit dem Druck in der Versorgungskammer In.O2 überein. Falls die Differenz zwischen dem Druck in der Steuerkammer In.K1 und dem Druck in der Versorgungskammer In.O2 oberhalb einer vorgegebenen oberen Schranke liegt, so kann das unerwünschte Ereignis aufgetreten sein, dass der Beutel 7 von innen gegen das Gehäuse 10 gepresst ist. Falls diese Druck-Differenz unterhalb einer vorgegebenen unteren Schranke liegt, so kann das unerwünschte Ereignis aufgetreten sein, dass der Beutel 7 stark oder sogar völlig zusammengedrückt ist. Falls eines dieser Ereignisse aufgetreten ist, so wird bevorzugt eine Meldung erzeugt und in einer von einem Menschen wahrnehmbaren Form ausgegeben.

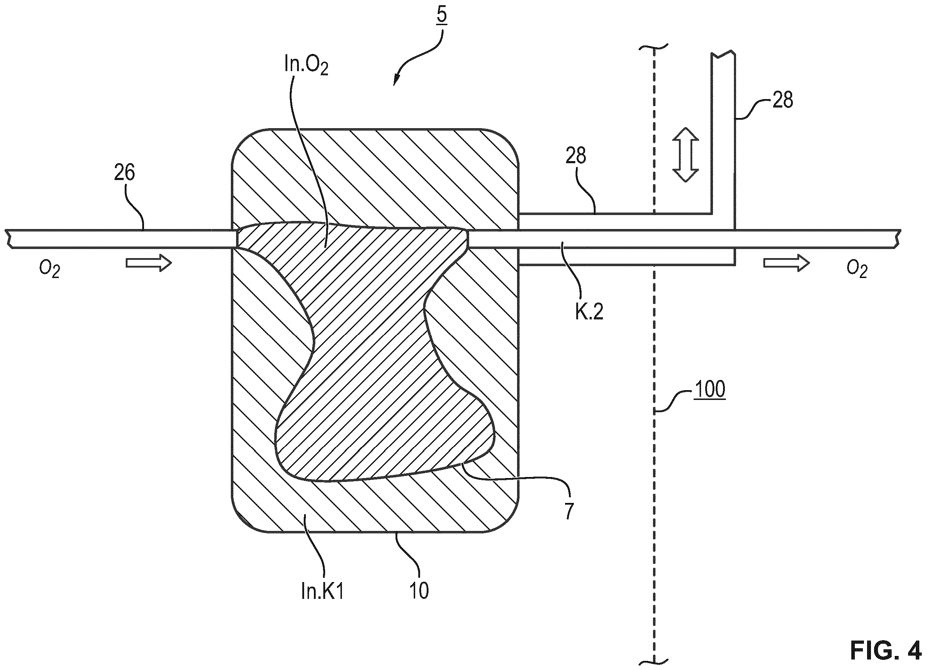

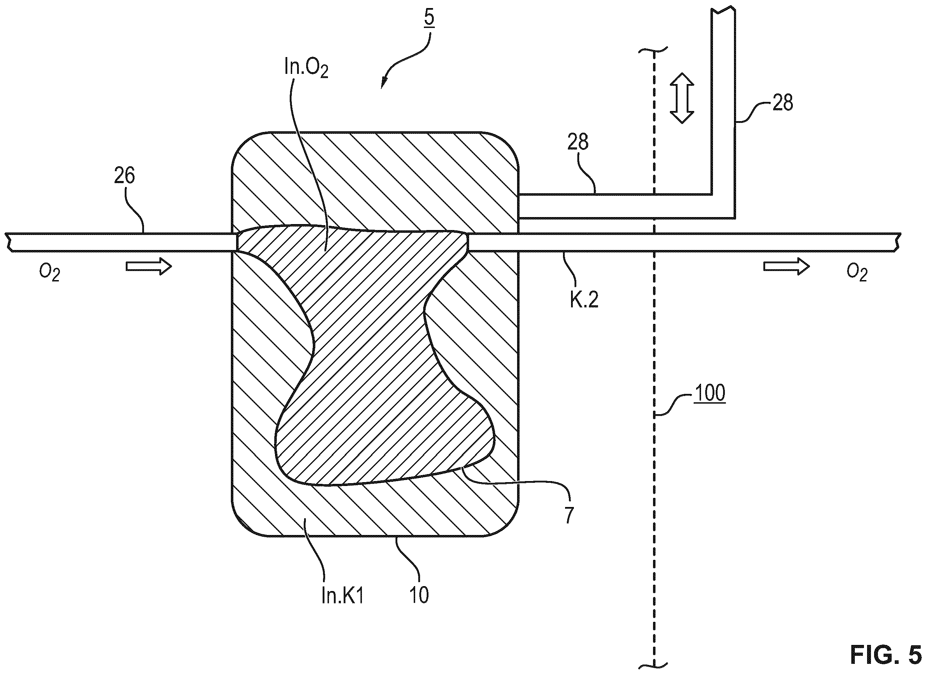

In einer Ausgestaltung ist der Pufferspeicher 5 ein Bestandteil des Beatmungsgeräts 100. In einer anderen Ausgestaltung lässt sich der Pufferspeicher 5 lösbar mit dem Beatmungsgerät 100 verbinden. Figur 4 und Figur 5 zeigen zwei mögliche Realisierungsformen, wie der Pufferspeicher 5 von außen lösbar mit dem Beatmungsgerät 100 verbunden wird. Gleiche Bezugszeichen haben wiederum die gleiche Bedeutung wie in Figur 1 und Figur 2.

Bei der Realisierungsform gemäß Figur 4 ist ein Abschnitt des zweiten Kanals K.2 koaxial durch das Innere eines Abschnitts der pneumatische Steuerleitung 28 hindurchgeführt. Daher lässt sich der Pufferspeicher 5 an einer einzigen pneumatischen Koppelstelle mit dem Beatmungsgerät 100 verbinden. Möglich ist auch, dass der Abschnitt der pneumatischen Steuerleitung 28 durch das Innere des Abschnitts des zweiten Kanals K.2 geführt ist.

Bei der Realisierungsform gemäß Figur 5 sind zwei räumlich voneinander beabstandete pneumatische Koppelstellen zwischen dem Pufferspeicher 5 und dem Beatmungsgerät 100 vorhanden, nämlich eine erste Koppelstelle für den zweiten Kanal K.2 und eine zweite Koppelstelle für die pneumatische Steuerleitung 28. Bevorzugt unterscheiden sich diese beiden Koppelstellen mechanisch voneinander, sodass eine mechanische Codierung bereitgestellt wird. Diese mechanische Codierung verhindert, dass der Pufferspeicher 5 falsch mit dem Beatmungsgerät 100 verbunden wird.

In der gerade beschriebenen Realisierungsform wird die Versorgungskammer In.O2 im Inneren des Beutels 7 gebildet, und die Steuerkammer In.K1 umgibt den Beutel 7. Möglich ist auch, dass umgekehrt die Steuerkammer In.K1 im Inneren des Beutels 7 gebildet wird und die Versorgungskammer In.O2 den Beutel 7 umgibt.

Figur 6 zeigt eine alternative Ausgestaltung des Trennelements. Anstelle eines Beutels 7 ist im Inneren des Gehäuses 10 eine flexible Membrane 7.1 befestigt. Diese Membrane 7.1 unterteilt den Innenraum im Gehäuse 10 in die Versorgungskammer In.O2 (in Figur 6 unten) und die Steuerkammer In.K1 (in Figur 6 oben). Die gestrichelten Linien zeigen zwei mögliche Auslenkungen der Membrane 7.1 nach oben und nach unten an. Möglich ist auch, dass sich die Versorgungskammer In.O2 oben und die Steuerkammer In.K1 unten befinden.

### Bezugszeichenliste

| | |
|---|---|
| 1 | pneumatischer Druckminderer im zweiten Kanal K.2, umfasst den Vordruck-Eingang V.3, den Hinterdruck-Ausgang V.2 und den Steuerdruck-Eingang V.4 |
| 2 | Gebläse des Beatmungsgeräts 100, ist über einen Versorgungs-Ausgang mit dem ersten Kanal K.1 verbunden, mit dem Einlass E verbunden, fungiert als die erste Quelle |
| 3 | signalverarbeitendes Steuergerät, erhält Messwerte von den Volumenfluss-Sensoren 6.1 und 6.2, steuert die Proportionalventile 4.1 und 4.2 an |
| 4.1 | Proportionalventil im ersten Kanal K.1, vermag den Volumenfluss durch den ersten Kanal K.1 zu verändern, vom Steuergerät 3 angesteuert |
| 4.2 | Proportionalventil im vierten Kanal K.4 oder im zweiten Kanal K.2, vermag den Volumenfluss durch den vierten Kanal K.4 oder den zweiten Kanal K.2 zu verändern, vom Steuergerät 3 angesteuert |
| 5 | Pufferspeicher, umfasst den Beutel 7 und das Gehäuse 10, bildet im Inneren des Gehäuses 10 die Steuerkammer In.K1 und die Versorgungskammer In.O2 aus |
| 6.1 | Volumenfluss-Sensor, misst ein Maß für den Volumenfluss durch den ersten Kanal K.1 |
| 6.2 | Volumenfluss-Sensor, misst ein Maß für den Volumenfluss durch den vierten Kanal K.4 oder den zweiten Kanal K.2 |
| 7 | flexibler Beutel im Inneren des Pufferspeichers 5, von der Steuerkammer In.K1 umgeben, umgibt die Versorgungskammer In.O2, mit den Leitungen 26 und 28 verbunden |
| 7.1 | flexible Membrane im Inneren des Pufferspeichers 5, trennt die Versorgungskammer In.O2 von der Steuerkammer In.K1 |
| 8 | Mischpunkt, in den die beiden Kanäle K.1 und K.3 oder K.2 münden |
| 9 | patientenseitige Koppeleinheit, mit dem Inspirations-Kanal K.30 verbunden |
| 10 | starres Gehäuse des Pufferspeichers 5 |
| 16.1 | Drucksensor, misst den Druck in der pneumatischen Steuerleitung 28 |
| 16.2 | Drucksensor, misst den Druck in dem zweiten Kanal K.2 |
| 18 | Mischpunkt, in dem der zweite Kanal K.2 in den dritten Kanal K.3 mündet und in dem der Inspirations-Kanal K.30 beginnt |
| 20 | stationärer Versorgungsanschluss in der Wand W für reinen Sauerstoff, mit dem dritten Kanal K.3 verbunden, fungiert als weitere Quelle |
| 21 | Versorgungsleitung, führt vom Versorgungsanschluss 20 zur Vordruck-Kammer Ka.1 im Druckminderer 1 |
| 23 | Filter hinter dem Einlass E |
| 25 | mobile Quelle für reinen Sauerstoff, mit dem zweiten Kanal K.2 verbunden, fungiert als zweite Quelle |
| 26 | Verbindungsleitung von der mobilen Quelle 25 zum Pufferspeicher 5 |
| 27 | Überdruckventil in der Verbindungsleitung 26 |
| 28 | pneumatische Steuerleitung, verbindet die Steuerkammer In.K1 mit dem ersten Kanal K.1 |
| 28.1 | weitere pneumatische Steuerleitung, verbindet den ersten Kanal K.1 mit dem Steuerdruck-Eingang V.4 |
| 29 | Rückschlagventil in der Versorgungsleitung 21 |
| 30 | Drehknopf, den ein Benutzer drehen kann, um den geforderten Anteil von Sauerstoff in dem Gasgemisch, das zur patientenseitigen Koppeleinheit 9 gefördert wird, vorzugeben |
| 32 | Spannungsversorgungseinheit des Beatmungsgeräts 100 |
| 100 | Beatmungsgerät, erzeugt ein Gasgemisch aus Atemluft und reinem Sauerstoff, beatmet den Patienten Pt künstlich, umfasst die erfindungsgemäße Versorgungs-Anordnung |
| E | Einlass für Umgebungsluft, fungiert als erste Quelle, mit dem Gebläse 2 verbunden |
| In.K1 | Steuerkammer im Inneren des Gehäuses 10, steht über die Steuerleitung 28 in einer Steuer-Fluidverbindung mit dem ersten Kanal K.1, umgibt in einer Ausgestaltung den Beutel 7 |
| In.O2 | Versorgungskammer im Inneren des Gehäuses 10, steht in jeweils einer Fluidverbindung mit der Verbindungsleitung 26 und in einer Versorgungs-Fluidverbindung mit dem zweiten Kanal K.2 |
| K.1 | erster Kanal, stellt Atemluft bereit, welches das Gebläse 2 fördert |
| K.2 | zweiter Kanal, stellt reinen Sauerstoff mit einem niedrigeren Druck aus der Quelle 25 bereit |
| K.3 | dritter Kanal, stellt reinen Sauerstoff mit einem höheren Druck aus dem Versorgungsanschluss 20 bereit, führt zum Mischpunkt 18 |
| K.4 | vierter Kanal, leitet reinen Sauerstoff vom Mischpunkt 18 zum Mischpunkt 8 |
| K.30 | Inspirations-Kanal, leitet das Gasgemisch vom Mischpunkt 8 zur patientenseitigen Koppeleinheit 9 |
| P | Druck, insbesondere durch den Inspirations-Kanal K.30 zur patientenseitigen Koppeleinheit 9 |
| Pt | Patient, wird vom Beatmungsgerät 100 künstlich beatmet, trägt die patientenseitige Koppeleinheit 9 |
| V.1 | Versorgungs-Verbindungselement des ersten Kanals K.1, mit einem Versorgungs-Ausgang des Gebläses 2 verbunden |
| V.2 | Hinterdruck-Ausgang des Druckminderers 1, mit dem zweiten Kanal K.2 verbunden |
| V.3 | Vordruck-Eingang des Druckminderers 1, mit der Versorgungsleitung 21 verbunden |
| V.4 | Steuerdruck-Eingang des Druckminderers 1, mit der weiteren pneumatischen Steuerleitung 28.1 verbunden |
| Vol' | Volumenfluss durch den Inspirations-Kanal K.30 zur patientenseitigen Koppeleinheit 9 |
| W | Wand, hat den Versorgungsanschluss 20 für reinen Sauerstoff |

## Patentansprüche

1. Versorgungs-Anordnung zur Versorgung einer patientenseitigen Koppeleinheit (9)
mit einem Gasgemisch umfassend einen ersten Gas-Bestandteil (Luft) und einen zweiten Gas-Bestandteil (O2),
wobei die patientenseitige Koppeleinheit (9) mit einem Patienten (Pt) verbunden oder verbindbar ist,
wobei die Versorgungs-Anordnung
- einen ersten Kanal (K.1) mit einem Versorgungs-Verbindungselement (V.1),
- einen zweiten Kanal (K.2),
- einen Mischpunkt (8),
- einen Inspirations-Kanal (K.30),
- einen Pufferspeicher (5) und
- eine pneumatische Steuerleitung (28)
umfasst,
wobei der Pufferspeicher (5) ein Gehäuse (10) und ein fluiddichtes Trennelement (7, 7.1) im Inneren des Gehäuses (10) umfasst,
wobei das Trennelement (7, 7.1) das Innere des Gehäuses (10) fluiddicht in eine Versorgungskammer (In.O2) und eine Steuerkammer (In.K1) unterteilt,
wobei eine Fluidverbindung zwischen dem Versorgungs-Verbindungselement (V.1) und einer ersten Quelle (E), nämlich einer Quelle für den ersten Gas-Bestandteil (Luft), hergestellt oder herstellbar ist,
wobei eine Fluidverbindung (26) zwischen der Versorgungskammer (In.O2) und einer zweiten Quelle (25), nämlich einer Quelle für den zweiten Gas-Bestandteil (O2), hergestellt oder herstellbar ist,
wobei eine Versorgungs-Fluidverbindung zwischen der Versorgungskammer (In.O2) und dem zweiten Kanal (K.2) hergestellt oder herstellbar ist,
wobei der erste Kanal (K.1) dazu ausgestaltet ist, den ersten Gas-Bestandteil (Luft) vom Versorgungs-Verbindungselement (V.1) zum Mischpunkt (8) zu leiten,
wobei der zweite Kanal (K.2) dazu ausgestaltet ist, den zweiten Gas-Bestandteil (O2) von der Versorgungskammer (In.O2) zum Mischpunkt (8) zu leiten,
wobei der Inspirations-Kanal (K.30) dazu ausgestaltet ist, ein im Mischpunkt (8) erzeugtes Gasgemisch zu der patientenseitigen Koppeleinheit (9) zu leiten,
wobei die pneumatische Steuerleitung (28) eine Steuer-Fluidverbindung zwischen dem ersten Kanal (K.1) und dem Pufferspeicher (5) herstellt,
wobei die Steuer-Fluidverbindung, welche die Steuerleitung (28) herstellt,
- die Steuerkammer (In.K1) mit dem ersten Kanal (K.1) verbindet und
- einen Druckausgleich zwischen der Steuerkammer (In.K1) und dem ersten Kanal (K.1) bewirkt, und
wobei die Versorgungs-Anordnung so ausgestaltet ist, dass der Pufferspeicher (5), die Versorgungs-Fluidverbindung und die Steuer-Fluidverbindung einen Druckausgleich zwischen
- dem Druck in der Versorgungskammer (In.O2),
- dem Druck im ersten Kanal (K.1) und
- dem Druck im zweiten Kanal (K.2)
bewirken,
**dadurch gekennzeichnet, dass**
das Trennelement (7, 7.1) als ein flexibler Beutel (7) ausgestaltet ist oder einen flexiblen Beutel (7) umfasst,
wobei zwischen dem Gehäuse (10) und dem Beutel (7) ein Innenraum gebildet wird, der den Beutel (7) umgibt,
wobei
- die Versorgungskammer (In.O2) im Inneren des Beutels (7) und die Steuerkammer (In.K1) im Zwischenraum zwischen dem Gehäuse (10) und dem Beutel (7) gebildet werden oder
- die Steuerkammer (In.K1) im Inneren des Beutels (7) und die Versorgungskammer (In.O2) im Zwischenraum zwischen dem Gehäuse (10) und dem Beutel (7) gebildet werden.

2. Versorgungs-Anordnung nach Anspruch 1,
**dadurch gekennzeichnet, dass**
die Versorgungskammer (In.O2) im Inneren des Beutels (7) gebildet wird und
- sowohl die Fluidverbindung (26) zwischen der Versorgungskammer (In.O2) und der zweiten Quelle (25)
- als auch die Versorgungs-Fluidverbindung
durch das Gehäuse (10) hindurchgeführt sind.

3. Versorgungs-Anordnung nach einem der vorhergehenden Ansprüche,
**dadurch gekennzeichnet, dass**
die Versorgungs-Anordnung eine Sensor-Anordnung (16.1, 16.2) umfasst,
wobei die Sensor-Anordnung (16.1, 16.2) dazu ausgestaltet ist, ein Maß für die Differenz zwischen
- dem Druck in der Versorgungskammer (In.O2) und
- dem Druck in der Steuerkammer (In.K1)
zu messen, und
wobei die Versorgungs-Anordnung dazu ausgestaltet ist, eine Meldung zu generieren, wenn die gemessene Druck-Differenz außerhalb eines vorgegebenen Bereichs liegt.

4. Versorgungs-Anordnung nach einem der vorhergehenden Ansprüche,
**dadurch gekennzeichnet, dass**
die Versorgungs-Anordnung
- einen dritten Kanal (K.3) und
- einen Druckminderer (1) mit einem Vordruck-Eingang (V.3) und einem Hinterdruck-Ausgang (V.2)
umfasst,
wobei eine Fluidverbindung (21) zwischen dem Vordruck-Eingang (V.3) des Druckminderers (1) und einer weiteren Quelle (20) für den zweiten Gas-Bestandteil (O2) oder einer Quelle für einen dritten Gas-Bestandteil hergestellt oder herstellbar ist,
wobei der Hinterdruck-Ausgang (V.2) des Druckminderers (1) mit dem dritten Kanal (K.3) verbunden ist,
wobei der dritte Kanal (K.3) dazu ausgestaltet ist, den zweiten Gas-Bestandteil (O2) oder den dritten Gas-Bestandteil von dem Hinterdruck-Ausgang (V.2) zu dem Mischpunkt (8) oder zu einem weiteren Mischpunkt (18) zu leiten,
wobei der weitere Mischpunkt (18) in einer Fluidverbindung (Ka.2) mit dem Mischpunkt (8) und / oder mit der patientenseitigen Koppeleinheit (9) steht und
wobei der Druckminderer (1) dazu ausgestaltet ist, den zweiten Gas-Bestandteil (O2) dergestalt bereitzustellen, dass der zeitliche Verlauf des Drucks am Hinterdruck-Ausgang (V.2) dem zeitlichen Verlauf des Drucks im ersten Kanal (K.1) und / oder den zeitlichen Verlauf des Drucks im zweiten Kanal (K.2) folgt.

5. Versorgungs-Anordnung nach Anspruch 4,
**dadurch gekennzeichnet, dass**
die Versorgungs-Anordnung eine weitere pneumatische Steuerleitung (28.1) umfasst und
der Druckminderer (1) einen Steuerdruck-Eingang (V.4) umfasst,
wobei die weitere pneumatische Steuerleitung (28.1) eine weitere Steuer-Fluidverbindung zwischen dem ersten Kanal (K.1) und dem Steuerdruck-Eingang (V.4) herstellt.

6. Versorgungs-System zur Versorgung einer patientenseitigen Koppeleinheit (9)
mit einem Gasgemisch umfassend einen ersten Gas-Bestandteil (Luft) und einen zweiten Gas-Bestandteil (O2),
wobei das Versorgungs-System
- eine erste Quelle (E),
- eine zweite Quelle (20) und
- eine Versorgungs-Anordnung nach einem der vorhergehenden Ansprüche
umfasst,
wobei die erste Quelle (E) dazu ausgestaltet ist, den ersten Gas-Bestandteil (Luft) bereitzustellen,
wobei die zweite Quelle (25) dazu ausgestaltet ist, den zweiten Gas-Bestandteil (O2) bereitzustellen,
wobei wenigstens zeitweise eine Fluidverbindung zwischen dem Versorgungs-Verbindungselement (V.1) der Versorgungs-Anordnung und der ersten Quelle (E) hergestellt ist und
wobei wenigstens zeitweise eine Fluidverbindung (26) zwischen der Versorgungskammer (In.O2) und der zweiten Quelle (25) für den zweiten Gas-Bestandteil (O2) hergestellt ist.

7. Versorgungs-System nach Anspruch 6,
**dadurch gekennzeichnet, dass**
das Versorgungs-System eine weiteren Quelle (20) für den zweiten Gas-Bestandteil (O2) oder für einen dritten Gas-Bestandteil umfasst und
die Versorgungs-Anordnung nach Anspruch 4 oder Anspruch 5 ausgestaltet ist,
wobei wenigstens zeitweise eine Fluidverbindung (21) zwischen dem Vordruck-Eingang (V.3) des Druckminderers (1) und der weiteren Quelle (20) hergestellt ist.

8. Versorgungs-System nach Anspruch 7,
**dadurch gekennzeichnet, dass**
die weitere Quelle (20) den zweiten Gas-Bestandteil (O2) mit einem höheren Druck bereitstellt als die zweite Quelle (25).

9. Beatmungs-System (100) zur künstlichen Beatmung eines Patienten (Pt),
wobei das Beatmungs-System (100)
- eine patientenseitige Koppeleinheit (9),
- eine Fluidfördereinheit (2) und
- eine Versorgungs-Anordnung nach einem der Ansprüche 1 bis 5 oder ein Versorgungs-System nach einem der Ansprüche 6 bis 8
umfasst,
wobei die patientenseitige Koppeleinheit (9) mit einem Patienten (Pt) verbunden oder verbindbar ist und
wobei die Fluidfördereinheit (2) dazu ausgestaltet ist, ein von der Versorgungs-Anordnung erzeugtes Gasgemisch zu der patientenseitigen Koppeleinheit (9) zu fördern.

## Claims

1. Supply arrangement for supplying a patient-side coupling unit (9)
with a gas mixture comprising a first gas component (air) and a second gas component (O2),
the patient-side coupling unit (9) being connected or connectable to a patient (Pt),
the supply arrangement comprising
- a first channel (K.1) comprising a supply connection element (V.1),
- a second channel (K.2),
- a mixing point (8),
- an inspiratory channel (K.30),
- a buffer tank (5) and
- a pneumatic control line (28),
the buffer tank (5) comprising a housing (10) and a fluid-tight separating element (7, 7.1) in the interior of the housing (10),
the separating element (7, 7.1) dividing the interior of the housing (10) in a fluid-tight manner into a supply chamber (In.O2) and a control chamber (In.K1),
a fluid connection being established or establishable between the supply connection element (V.1) and a first source (E), namely a source of the first gas component (air),
a fluid connection (26) being established or establishable between the supply chamber (In.O2) and a second source (25), namely a source of the second gas component (O2),
a supply fluid connection being established or establishable between the supply chamber (In.O2) and the second channel (K.2),
the first channel (K.1) being configured to conduct the first gas component (air) from the supply connection element (V.1) to the mixing point (8),
the second channel (K.2) being configured to conduct the second gas component (O2) from the supply chamber (In.O2) to the mixing point (8),
the inspiratory channel (K.30) being configured to conduct a gas mixture generated in the mixing point (8) to the patient-side coupling unit (9),
the pneumatic control line (28) establishing a control fluid connection between the first channel (K.1) and the buffer tank (5),
the control fluid connection which the control line (28) establishes
- connecting the control chamber (In.K1) with the first channel (K.1) and
- effecting a pressure equalization between the control chamber (In.K1) and the first channel (K.1), and
the supply arrangement being configured such that the buffer tank (5), the supply fluid connection and the control fluid connection effect a pressure equalization between
- the pressure in the supply chamber (In.O2),
- the pressure in the first channel (K.1) and
- the pressure in the second channel (K.2),
**characterized in that**
the separating element (7, 7.1) is configured as a flexible bag (7) or comprises a flexible bag (7),
an interior space being formed between the housing (10) and the bag (7), which interior space surrounds the bag (7),
- the supply chamber (In.O2) being formed in the interior of the bag (7) and the control chamber (In.K1) being formed in the space between the housing (10) and the bag (7) or
- the control chamber (In.K1) being formed in the interior of the bag (7) and the supply chamber (In.O2) being formed in the space between the housing (10) and the bag (7).

2. Supply arrangement according to claim 1,
**characterized in that**
the supply chamber (In.O2) is formed in the interior of the bag (7) and
- both the fluid connection (26) between the supply chamber (In.O2) and the second source (25)
- and the supply fluid connection
are passed through the housing (10).

3. Supply arrangement according to any of the preceding claims,
**characterized in that**
the supply arrangement comprises a sensor arrangement (16.1, 16.2),
the sensor arrangement (16.1, 16.2) being configured to measure the difference between
- the pressure in the supply chamber (In.O2) and
- the pressure in the control chamber (In.K1),
and
the supply arrangement being configured to generate a message if the measured pressure difference is outside a predetermined range.

4. Supply arrangement according to any of the preceding claims,
**characterized in that**
the supply arrangement comprises
- a third channel (K.3) and
- a pressure regulator (1) comprising an upstream pressure inlet (V.3) and a downstream pressure outlet (V.2),
a fluid connection (21) being established or establishable between the upstream pressure inlet (V.3) of the pressure regulator (1) and a further source (20) of the second gas component (O2) or a source of a third gas component,
the downstream pressure outlet (V.2) of the pressure regulator (1) being connected to the third channel (K.3),
the third channel (K.3) being configured to conduct the second gas component (O2) or the third gas component from the downstream pressure outlet (V.2) to the mixing point (8) or to a further mixing point (18),
the further mixing point (18) being in a fluid connection (Ka.2) with the mixing point (8) and/or with the patient-side coupling unit (9), and
the pressure regulator (1) being configured to provide the second gas component (O2) in such a way that the variation over time of the pressure at the downstream pressure outlet (V.2) follows the variation over time of the pressure in the first channel (K.1) and/or the variation over time of the pressure in the second channel (K.2).

5. Supply arrangement according to claim 4,
**characterized in that**
the supply arrangement comprises a further pneumatic control line (28.1) and
the pressure regulator (1) comprises a control pressure inlet (V.4),
the further pneumatic control line (28.1) establishing a further control fluid connection between the first channel (K.1) and the control pressure inlet (V.4).

6. Supply system for supplying a patient-side coupling unit (9)
with a gas mixture comprising a first gas component (air) and a second gas component (O2),
the supply system comprising
- a first source (E),
- a second source (20) and
- a supply arrangement according to any of the preceding claims,
the first source (E) being configured to provide the first gas component (air),
the second source (25) being configured to provide the second gas component (O2),
a fluid connection being at least temporarily established between the supply connection element (V.1) of the supply arrangement and the first source (E), and
a fluid connection (26) being at least temporarily established between the supply chamber (In.O2) and the second source (25) of the second gas component (O2).

7. Supply system according to claim 6,
**characterized in that**
the supply system comprises a further source (20) of the second gas component (O2) or of a third gas component and
the supply arrangement is configured according to claim 4 or claim 5,
a fluid connection (21) being at least temporarily established between the upstream pressure inlet (V.3) of the pressure regulator (1) and the further source (20).

8. Supply system according to claim 7,
**characterized in that**
the further source (20) provides the second gas component (O2) at a higher pressure than the second source (25).

9. Ventilation system (100) for artificial ventilation of a patient (Pt), the ventilation system (100) comprising
- a patient-side coupling unit (9),
- a fluid conveying unit (2) and
- a supply arrangement according to any of claims 1 to 5 or a supply system according to any of claims 6 to 8,
the patient-side coupling unit (9) being connected or connectable to a patient (Pt) and
the fluid conveying unit (2) being configured to convey a gas mixture generated by the supply arrangement to the patient-side coupling unit (9).

## Revendications

1. Agencement d'alimentation permettant d'alimenter une unité de couplage (9) côté patient
comportant un mélange de gaz comprenant un premier constituant gazeux (air) et un deuxième constituant gazeux (O2),
dans lequel l'unité de couplage (9) côté patient est reliée ou peut être reliée à un patient (Pt),
dans lequel l'agencement d'alimentation comprend
- un premier canal (K.1) comportant un élément de liaison d'alimentation (V.1),
- un deuxième canal (K.2),
- un emplacement de mélange (8),
- un canal d'inspiration (K.30),
- un réservoir tampon (5) et
- une conduite de commande (28) pneumatique,
dans lequel le réservoir tampon (5) comprend un boîtier (10) et un élément de séparation (7, 7.1) étanche aux fluides à l'intérieur du boîtier (10),
dans lequel l'élément de séparation (7, 7.1) divise l'intérieur du boîtier (10) de manière étanche aux fluides en une chambre d'alimentation (In.O2) et en une chambre de commande (In.K1),
dans lequel une liaison fluidique est établie ou peut être établie entre l'élément de liaison d'alimentation (V.1) et une première source (E), à savoir une source pour le premier constituant gazeux (air),
dans lequel une liaison fluidique (26) est établie ou peut être établie entre la chambre d'alimentation (In.O2) et une deuxième source (25), à savoir une source pour le deuxième constituant gazeux (O2),
dans lequel une liaison fluidique d'alimentation est établie ou peut être établie entre la chambre d'alimentation (In.O2) et le deuxième canal (K.2),
dans lequel le premier canal (K.1) est conçu pour conduire le premier constituant gazeux (air) depuis l'élément de liaison d'alimentation (V.1) jusqu'à l'emplacement de mélange (8),
dans lequel le deuxième canal (K.2) est conçu pour conduire le deuxième constituant gazeux (O2) depuis la chambre d'alimentation (In.O2) jusqu'à l'emplacement de mélange (8),
dans lequel le canal d'inspiration (K.30) est conçu pour conduire un mélange de gaz généré à l'emplacement de mélange (8) vers l'unité de couplage (9) côté patient,
dans lequel la conduite de commande pneumatique (28) établit une liaison fluidique de commande entre le premier canal (K.1) et le réservoir tampon (5),
dans lequel la liaison fluidique de commande qui établit la conduite de commande (28)
- relie la chambre de commande (In.K1) au premier canal (K.1) et
- provoque une compensation de pression entre la chambre de commande (In.K1) et le premier canal (K.1), et
dans lequel l'agencement d'alimentation est conçu de sorte que le réservoir tampon (5), la liaison fluidique d'alimentation et la liaison fluidique de commande provoquent une compensation de pression entre
- la pression dans la chambre d'alimentation (In.O2),
- la pression dans le premier canal (K.1) et
- la pression dans le deuxième canal (K.2),
**caractérisé en ce que**
l'élément de séparation (7, 7.1) est conçu comme un sachet (7) flexible ou comprend un sachet (7) flexible,
dans lequel un espace intérieur est formé entre le boîtier (10) et le sachet (7), lequel espace intérieur entoure le sachet (7),
dans lequel
- la chambre d'alimentation (In.O2) est formée à l'intérieur du sachet (7) et la chambre de commande (In.K1) est formée dans l'espace intermédiaire entre le boîtier (10) et le sachet (7) ou
- la chambre de commande (In.K1) est formée à l'intérieur du sachet (7) et la chambre d'alimentation (In.O2) est formée dans l'espace intermédiaire entre le boîtier (10) et le sachet (7).

2. Agencement d'alimentation selon la revendication 1,
**caractérisé en ce que**
la chambre d'alimentation (In.O2) est formée à l'intérieur du sachet (7) et
- à la fois la liaison fluidique (26) entre la chambre d'alimentation (In.O2) et la deuxième source (25)
- et la liaison fluidique d'alimentation
sont guidées à travers le boîtier (10).

3. Agencement d'alimentation selon l'une des revendications précédentes,
**caractérisé en ce que**
l'agencement d'alimentation comprend un agencement de capteurs (16.1, 16.2),
dans lequel l'agencement de capteurs (16.1, 16.2) est configuré pour mesurer une mesure pour la différence entre
- la pression dans la chambre d'alimentation (In.O2) et
- la pression dans la chambre de commande (In.K1)
et
dans lequel l'agencement d'alimentation est configuré pour générer un message lorsque la différence de pression mesurée se situe en dehors d'une plage prédéterminée.

4. Agencement d'alimentation selon l'une des revendications précédentes,
**caractérisé en ce que**
l'agencement d'alimentation comprend
- un troisième canal (K.3) et
- un détendeur (1) comportant une entrée de pression amont (V.3) et une sortie de pression aval (V.2),
dans lequel une liaison fluidique (21) est établie ou peut être établie entre l'entrée de pression amont (V.3) du détendeur (1) et une autre source (20) pour le deuxième constituant gazeux (O2) ou une source pour un troisième constituant gazeux,
dans lequel la sortie de pression aval (V.2) du détendeur (1) est reliée au troisième canal (K.3),
dans lequel le troisième canal (K.3) est conçu pour conduire le deuxième constituant gazeux (O2) ou le troisième constituant gazeux depuis la sortie de pression aval (V.2) vers l'emplacement de mélange (8) ou vers un autre emplacement de mélange (18),
dans lequel l'autre emplacement de mélange (18) est en liaison fluidique (Ka.2) avec l'emplacement de mélange (8) et/ou avec l'unité de couplage (9) côté patient et
dans lequel le détendeur (1) est conçu pour fournir le deuxième constituant gazeux (O2) de telle sorte que l'évolution temporelle de la pression à la sortie de pression aval (V.2) suit l'évolution temporelle de la pression dans le premier canal (K.1) et/ou l'évolution temporelle de la pression dans le deuxième canal (K.2).

5. Agencement d'alimentation selon la revendication 4,
**caractérisé en ce que**
l'agencement d'alimentation comprend une autre conduite de commande (28.1) pneumatique et
le détendeur (1) comprend une entrée de pression de commande (V.4),
dans lequel l'autre conduite de commande (28.1) pneumatique établit une autre liaison fluidique de commande entre le premier canal (K.1) et l'entrée de pression de commande (V.4).

6. Agencement d'alimentation permettant d'alimenter une unité de couplage (9) côté patient
comportant un mélange de gaz comprenant un premier constituant gazeux (air) et un deuxième constituant gazeux (O2),
dans lequel l'agencement d'alimentation comprend
- une première source (E),
- une deuxième source (20) et
- un agencement d'alimentation selon l'une des revendications précédentes,
dans lequel la première source (E) est conçue pour fournir le premier constituant gazeux (air),
dans lequel la deuxième source (25) est conçue pour fournir le deuxième constituant gazeux (O2),
dans lequel une liaison fluidique est établie au moins temporairement entre l'élément de liaison d'alimentation (V.1) de l'agencement d'alimentation et la première source (E) et
dans lequel une liaison fluidique (26) est établie au moins temporairement entre la chambre d'alimentation (In.O2) et la deuxième source (25) pour le deuxième constituant gazeux (O2).

7. Système d'alimentation selon la revendication 6,
**caractérisé en ce que**
le système d'alimentation comprend une autre source (20) pour le deuxième constituant gazeux (O2) ou pour un troisième constituant gazeux et
l'agencement d'alimentation est conçu selon la revendication 4 ou la revendication 5,
dans lequel une liaison fluidique (21) est établie au moins temporairement entre l'entrée de pression amont (V.3) du détendeur (1) et l'autre source (20).

8. Système d'alimentation selon la revendication 7,
**caractérisé en ce que**
l'autre source (20) fournit le deuxième constituant gazeux (O2) à une pression supérieure à celle de la deuxième source (25).

9. Système de ventilation (100) pour la ventilation artificielle d'un patient (Pt), dans lequel le système de ventilation (100) comprend
- une unité de couplage (9) côté patient,
- une unité de transport de fluide (2) et
- un agencement d'alimentation selon l'une des revendications 1 à 5 ou un système d'alimentation selon l'une des revendications 6 à 8,
dans lequel l'unité de couplage (9) côté patient est reliée ou peut être reliée à un patient (Pt) et
dans lequel l'unité de transport de fluide (2) est conçue pour transporter un mélange de gaz généré par l'agencement d'alimentation vers l'unité de couplage (9) côté patient.
